# EUROPEAN PATENT APPLICATION

(11) **EP 4 834 323 A1**
(43) Date of publication of application: **04.11.2026**
(21) Application number: 25173323.4
(22) Date of filing: 29.04.2025
(51) Int. Cl.: A61K 9/00, A61K 31/519, A61K 47/34, A61K 47/36, A61P 15/10, A61P 17/00, A61P 17/14

(54) **DISSOLVABLE MICRONEEDLE COMPRISING PLGA MICROPARTICLE-ENCAPSULATED DUAL MODE-OF-ACTION PDE5 INHIBITOR-ORGANIC NITRATE ESTERS**

(71) Applicant: University-Industry Foundation(UIF), Yonsei University, Seoul 03722 (KR); Juvic Inc., Seoul 08389 (KR); Topadur Pharma AG, 8952 Schlieren (CH)
(72) Inventor: HYUNGIL, Jung, 03722 Seoul (KR); SHIN, Jiwoo, 03722 Seoul (KR); KANG, Geonwoo, 08389 Seoul (KR); NAEF, Reto, 8952 Schlieren (CH); TENOR, Hermann, 8952 Schlieren (CH); LUDIN, Christian, 4101 Oberwil (CH); BRAUN, Tobias, 8952 Schlieren (CH)
(74) Representative: Latscha Schöllhorn Partner AG

(57) **Abstract**

The present invention relates to microneedles comprising PLGA microparticles, wherein said microparticles encapsulate compounds of Formula (I) as described herein. The compounds of Formula (I) are dual pharmacology NO-releasing PDE5 inhibitors. In preferred embodiments, the microneedles can be arranged in array patches and/or applied to a subject using an applicator. The microneedles can be used to treat diseases or disorders, and/or to prevent or treat hair loss or promote hair growth.

## Description

### Technical Field

The present invention relates to microneedles comprising PLGA microparticles, wherein said microparticles encapsulate compounds of Formula (I) as described herein. The compounds of Formula (I) are dual pharmacology NO-releasing PDE5 inhibitors. In preferred embodiments, the microneedles can be arranged in array patches and/or applied to a subject using an applicator. The microneedles can be used to treat diseases or disorders, and/or to prevent or treat hair loss or promote hair growth.

### Related Art

Phosphodiesterases (PDEs) are enzymes that catalyze the hydrolysis and thus the degradation of cyclic adenosine monophosphate (cAMP) and cyclic guanosine monophosphate (cGMP) and thereby regulate intracellular levels of second messengers. Inhibition of PDEs leads to increasing intracellular concentrations of endogenous cAMP/cGMP. Therefore, inhibition of PDE can mediate a variety of physiological mechanisms at different cell and organ levels. Phosphodiesterase type 5 (PDE5) hydrolyses cyclic guanylate monophosphate (cGMP) specifically to 5' GMP. The selective inhibition of PDE5 has been validated as a therapeutic approach and strategies directed to promote inhibition of PDE5 activity have been applied as therapeutic tools, in particular, in neuronal, vascular and cardiovascular diseases and disorders. Moreover, the introduction of PDE5 inhibitors has revolutionized the treatment of male erectile dysfunction (Andersson KE, British Journal of Pharmacology (2018) 175:2554-2565; Das A et al. Pharmacol Ther. (2015) 147:12-21; Dobhal T et al. Critical Review in Pharmaceutical Sciences (2012) 1(3):13-27).

Most prominent examples of PDE5 inhibitors are Sildenafil, Tadalafil and Vardenafil, which have been described among others, for example, in WO 99/24433, WO 01/60825, EP 995'751 and WO 2011/075655. Recently a novel class of PDE 5 inhibitors with dual-pharmacology releasing NO in addition to its PDE 5 inhibition in a more than additive fashion has been described in WO/2018/215433.

Hair is produced in hair follicles, which are located in the dermis layer above the subcutaneous fat below the epidermal layer of skin. Hair follicles are located on all parts of the body except the lips, palms and soles of the feet, and new hair is made from the hair matrix at the bottom of the hair follicles. The living cells in the hair matrix proliferate and push upward, and when these cells dry quickly and die, they are compressed into a dense and hard mass to form a hair shaft. The hair shaft, which is made of dead protein, is covered with a delicate layer (cuticula pili) composed of platelike scales.

The hair growth cycle consists of three phases: the anagen phase, where hair grows most actively, followed by the catagen phase, where hair degeneration begins, and the telogen phase, where hair growth is stopped. At the end of the telogen phase, the hair falls out and a new cycle begins again as new hair grows from the hair follicles. The anagen phase of eyebrows and eyelashes is about 1 to 6 months, and the anagen phase of hair (e.g., on the top of a human head) is about 2 to 6 years, and in general, about 50 to 100 hairs fall out at the end of the telogen phase each day.

The most common types of hair loss are male pattern baldness and alopecia. Many studies have been conducted to alleviate or treat alopecia, and in particular, research has been carried out in the cosmetic or pharmaceutical industry to develop new substances for stimulating hair growth or alleviating hair loss. Accordingly, drug therapy using, e.g., female hormones, blood flow promoters and hair transplantation have been developed. Currently, the most commonly used drugs for the treatment of hair loss are 2,4-diamino-6-piperidinopyrimidine-3-oxide (also known as minoxidil or by the trade name "Rogaine^{®}"; see e.g., US Patent Nos. 4,193,619 and 4,596,812), and finasteride (also known under the trade name "Propecia^{®}"), which is a specific inhibitor of type II 5α-reductase.

Minoxidil is a drug that induces hair growth by increasing blood flow through the vasodilating effect and supplying nutrients to the hair root. In particular, it is known to be effective for alleviating hair loss symptoms in the forehead area. Minoxidil is known to reduce hair loss by up to 10% and promote hair growth in men suffering from male pattern baldness, but it has to be applied directly to the scalp from the outside, it has to be used regularly for a long period of time, and it must be applied twice a day, thus limiting patient compliance. A further disadvantage that it does not exert a very good effect on hair loss in areas other than the forehead area.

Finasteride is commonly administered orally as a pill. It can reduce hair loss by inhibiting the function of type II 5α-reductase and preventing the conversion of testosterone to dihydrotestosterone (DHT). However, finasteride also requires continuous and regular administration, and for some patients, it has side effects such as decreased libido, erectile dysfunction and the like, and at it can be used only for adult men.

Various drug delivery methods have been developed in an attempt to more effectively deliver minoxidil and finasteride. For instance, Paredes *et al.* teach the use of microneedle array patches to deliver finasteride to patients. However, the patches needed to be applied every three days to enable significant hair growth (see Paredes et al., International Journal of Pharmaceutics, 606, p. 120885 (2001)).

Moreover, the use of conventional dissolvable microneedles (DMNs) for alopecia treatment is hindered by the fact that DMNs need to be applied directly to the scalp, which is characterized by the presence of hair. These hairs not only interfere with the insertion of the DMNs but also make it challenging to use conventional arrays of DMNs, which rely on sticky patches that provide the firm attachment to the skin necessary for transdermal drug delivery.

Candlelit microneedles (CMNs) are a type of dissolvable microneedle (DMN) that can reach deeper into the dermis layer than conventional conical DMNs and have been used for drug delivery ((Ohn, J. et al., (2021) Dissolving Candlelit Microneedle for Chronic Inflammatory Skin Diseases. Advanced Science, 8(14), 2004873).

Kim et al. teach the use of CMNs loaded with the dual-mode vasodilator M119 for the treatment of alopecia. However, even when using CMSs, Kim teaches that frequent administration (i.e., once every two days) was necessary in order to achieve improved hair growth indices compared to conventional DMNs and topical formulations (see Kim et al., Advanced Therapeutics, 5(8), p. 2200052 (2022)).

Thus, there remains a need for effective therapies for hair loss (e.g., caused by alopecia) that do not require such frequent dosing or administration.

### Summary of the Invention

It is an object of the present invention to treat skin a disease or disorder, prevent or treat hair loss and/or promote hair growth using a microneedle, preferably a dissolvable microneedle, that requires less frequent administration compared to previous methods currently known in the art.

The disclosed invention relates to microneedles (preferably dissolvable microneedles, more preferably dissolvable candlelit microneedles) comprising PLGA microparticles, wherein the microparticles comprise a compound of Formula (I). As described herein and as shown in the Examples, compounds of Formula (I) are preferably encapsulated in the PLGA microparticles. Administration of said PLGA microparticles via the dissolvable microneedles led to sustained release (e.g., over two weeks) of the compounds of Formula (I), as well as lactic acid (or lactate) and glycolic acid (or glycolate) from the PLGA microparticles after rapid (i.e., less than about 30 minutes) dissolution of the microneedles. The administration of said PLGA microparticles (encapsulating a compound of Formula (I)) via dissolvable microneedles led to increased hair growth efficacy as described herein compared to conventional hair growth agents.

For instance, use of a single administration of an array of dissolvable microneedles comprising PLGA microparticles as disclosed herein led to similar hair growth effects in a mouse model of alopecia as compared to the twice daily administration of minoxidil for 28 days, and with faster onset of hair growth. The sustained release of compounds of Formula (I) from the PLGA microparticles comprised in the microneedles disclosed herein also enables greater patient compliance because less frequent administration is necessary, thus leading to improved efficacy. Moreover, in preferred embodiments, administration of dissolvable microneedles, preferably candlelit dissolvable microneedles comprising PLGA microparticles encapsulating a compound of Formula (I) directly to the primary site of action allows application of efficacious therapeutic doses of compounds of Formula (I) to be significantly lower as compared to minoxidil. Without wising to be bound by theory, the low dose at the primary site of action is expected to result in fewer side effects of the inventive dissolvable microneedles comprising PLGA microparticles (e.g., blurred vision or other changes in vision, chest pain, dizziness, fainting, fast or irregular heartbeat in the case of minoxidil and systemic arterial hypotension in the case of oral PDE5 inhibitors).

Accordingly, the present disclosure relates to the use of PLGA microparticles to encapsulate a compound of Formula (I), enabling sustained release of compounds of Formula (I) to the skin of a subject; and to administration of the PLGA microparticles to the skin of a subject by dissolvable microneedles, preferably candlelit microneedles, to treat a disease or disorder (e.g., a skin disorder), to prevent or treat hair loss, and/or to promote hair growth (e.g., to treat alopecia). The sustained release of compounds of Formula (I) from the PLGA microparticles can lead to greater patient compliance by enabling an application rate of a single application per month (e.g., a single application once every 30 days). Through *in vitro* and *in vivo* experimentation, the examples herein demonstrate penetration of PLGA microparticle-containing dissolvable candlelit microneedles into animal model skin; and delivery of the PLGA microparticles encapsulating a compound of Formula (I) to the skin, e.g. to the dermis, preferably to hair follicles within the dermis for promoted hair regrowth. The inventive PLGA microparticle-containing dissolvable candlelit microneedles were able to penetrate the skin of a mouse model regardless of whether the mouse skin had previously been shaved, demonstrating that the inventive CMN arrays work both in the presence and absence of hair on the skin of a subject.

Without wising to be bound by theory, the present disclosure shows that the microneedles act as the tool of skin penetration and delivery, while the sustained release of compounds of Formula (I) is achieved through degradation of the PLGA microparticles encapsulating the compounds of Formula (I).

The dissolvable microneedles comprising PLGA microparticles encapsulating a compound of Formula (I) disclosed herein can overcome the limitation of previous hair growth treatments (e.g., alopecia treatments) which use conventional dissolvable microneedles. Such limitations include the need for frequent administration and low drug efficacy due to the skin barrier.

Additionally, the possibility of PLGA as a hair growth agent alone was examined. Administration of PLGA microparticles which did not include a compound of Formula (I) surprisingly led to appreciable hair regrowth, while inclusion of compounds of Formula (I) in the PLGA microparticles led to even greater hair regrowth compared to PLGA microparticles that did not comprise a compound of Formula (I). Additional features and advantages of the invention are described below.

### Description of Drawings

FIG 1 is a schematic drawing of a candlelit shaped microneedle (CMN) as used herein. As shown in FIG 1, a candlelit shaped microneedle comprises (i) an insertion layer (13), preferably wherein said insertion layer comprises: (a) a core layer (13a); and (b) a tip layer (13b); (ii) a base layer (12); wherein said base layer is attached to (iii) a substrate (11).

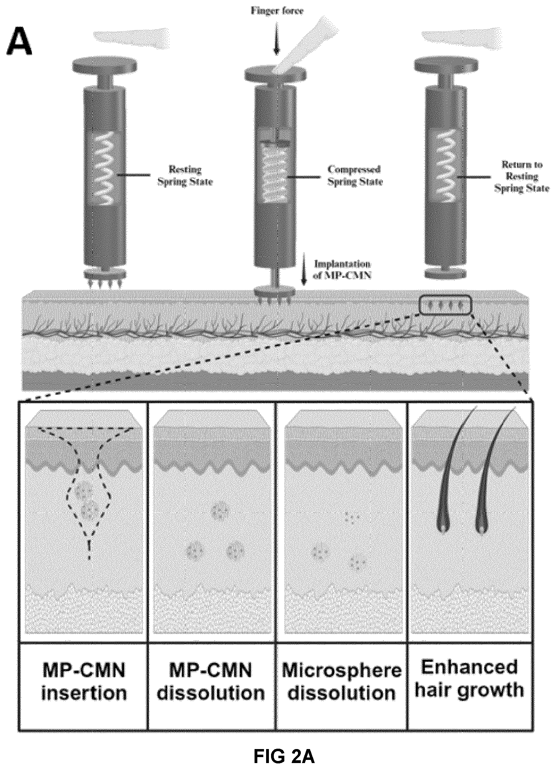

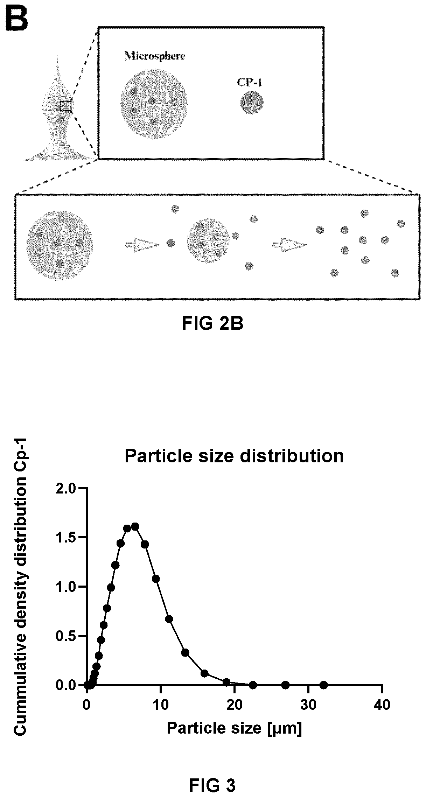

FIG 2 is a schematic of the procedure for increasing hair growth described herein. FIG 2A is a schematic of the insertion of dissolvable candlelit microneedles comprising PLGA microparticles using a spring-loaded applicator, which drives the microparticle-containing microneedles into the skin (e.g., through the stratum corneum) of a subject using the elastic force of the spring. FIG 2B is a schematic of the PLGA microparticle-containing candlelit microneedles. Each candlelit microneedle in the array contains microparticles encapsulating a compound of Formula (I) located in the core layer of the microneedle. When the candlelit microneedle dissolves, the PLGA microparticles containing a compound of Formula (I) are released.
FIG 3 is a plot of the particle size distribution of PLGA (50:50 lactic acid:glyclolic acid microparticles produced as described in the examples.
FIG 4 is a plot of the cumulative release profile of Compound 1 from PLGA microparticles into human blood plasma-SISF buffer solution over 29 days.
FIG 5A is a schematic of the preparation method for preparing candlelit microneedles as described in Example 5.
FIG 5B is a schematic image of cut dissolvable microneedle comprising PLGA microparticles.
FIG 6A shows a measurement of the fracture force of microneedle on MP-CMN array. (n=5).
FIG 6B shows optical coherence tomography (OCT) of MP-CMN inserted into porcine cadaver skin over time: (i) over 15 minutes after application with 5 minutes interval (ii) with white dashed outline of CMN geometry (scale bar: 500 µm).
FIG 7A is a schematic representation of the in vivo experimental protocol described in the examples for promoting hair regrowth.
FIG 7B is a group of representative photos of the dorsal skin area of mice from experimental groups on days 1, 7, 14, 21, and 28 of treatment as described in the examples.
FIG 7C is a plot of the percent hair coverage area of the dorsal skin area on days 0, 7, 14, 21 and 28 of treatment as described in the in vivo experimental protocol disclosed in the examples.
FIG 8A shows a schematic image of mouse skin lateral and longitudinal excision and a cross-sectional view for determining changes in skin thickness and hair thickness in the in vivo experimental protocol disclosed in the examples.
FIG 8B shows H&E staining results for both lateral and longitudinal sectional image of mouse dorsal skin (scale bar: 1000 µm) as described in the in vivo experimental protocol disclosed in the examples.
FIG 8C shows skin thickness measurements from mouse dorsal hair after day 28 as described in the in vivo experimental protocol disclosed in the examples.
FIG 8D shows the number of hairs per mm of both the lateral and the longitudinal sectional image as described in the in vivo experimental protocol disclosed in the examples.

### Detailed Description of the Invention

### Definitions

Unless defined otherwise, all technical and scientific terms used herein have the same meanings as commonly understood by one of ordinary skill in the art to which the present invention pertains. In general, the nomenclature used herein and the experimental methods described below are those well-known and commonly used in the art.

The term "a plurality of" is understood to mean "more than one." For instance, in preferred embodiments the microneedles of the present invention comprise more than one PLGA microparticle. A plurality of microparticles can encompass any number of microparticles greater than two.

The term "alkyl", as used herein, refers to a straight or branched hydrocarbon chain radical consisting solely of carbon and hydrogen atoms, containing no unsaturation, having typically and preferably from one to six carbon atoms (*e.g*., (C₁-₆alkyl), and which typically is attached to the rest of the molecule by a single bond. Whenever it appears herein, a numerical range such as "1 to 6" refers to each integer in the given range. For example, "1 to 6 carbon atoms" means that the alkyl group may consist of 1 carbon atom, 2 carbon atoms, 3 carbon atoms, *etc.,* up to and including 6 carbon atoms, although the definition is also intended to cover the occurrence of the term "alkyl" where no numerical range is specifically designated. Typical alkyl groups include, but are not limited to methyl, ethyl, *n*-propyl, prop-2-yl, *n*-butyl, but-2-yl, 2-methyl-prop-1-yl or 2-methyl-prop-2-yl.

As used herein, the term "treatment" refers to therapeutic treatment or a prophylactic or preventive measure to prevent or ameliorate a targeted pathological condition or disorder. Thus, in one aspect, treatment may include directly acting on, curing, inhibiting, ameliorating, impeding, preventing, reducing the severity, delaying the onset, or alleviating the symptoms associated with the disease, disorder or condition, or a combination thereof. That is, in one aspect, the term "treatment" means delaying progression, promoting a decline, inducing a decline, enhancing a decline, promoting recovery, increasing efficacy or decreasing resistance to an alternative therapeutic agent or a combination thereof. In one aspect, the term "prevention" refers to delaying the onset of symptoms, preventing recurrences, reducing the frequency or number of recurrences, increasing the latency between symptom occurrences or a combination thereof. In one embodiment, the term "inhibition" or "impediment" refers to alleviating the severity of symptoms, alleviating the severity of the current episode, reducing the number of symptoms, reducing the incidence of symptoms, reducing the latency of symptoms, alleviating symptoms, reducing secondary symptoms, reducing secondary infections or a combination thereof. In preferred embodiments, the term "amelioration" refers to any action that at least reduces a parameter, for example, the severity of a symptom, related to the treated state of a disease by administration of a composition including the compound.

In one aspect, the symptom is primary, and in another aspect, the symptom is secondary. As used herein, the term "primary" refers to a symptom that is a direct result of a disease, e.g., alopecia, and in one aspect, the term "secondary" refers to a symptom that results from or is a result of a primary cause of the disease. In preferred embodiments, the dissolvable microneedles described herein can be used to treat the primary and/or secondary symptoms of a disease, e.g., alopecia or seborrheic dermatitis.

In another aspect, the term "symptom" may be any symptom of hair loss, including hair loss, baldness, temporary hair loss, patchy hair loss, degenerative skin disorders or a combination thereof.

As used herein, the term "effective amount" refers to an amount necessary or sufficient to implement a desired biological effect. Preferably, the term "effective amount" refers to an amount of the compound of Formula (I) of the present invention, which is capable of (i) treating or preventing a particular disease, medical condition, or disorder, (ii) weakening, alleviating or eliminating one or more symptoms of the particular disease, medical condition, or disorder, or (iii) preventing or delaying the onset of one or more symptoms of the particular disease, medical condition, or disorder described in the present specification. An effective amount of the compound of the present invention of Formula (I) or the pharmaceutical composition will be an amount that achieves this selected result, and such amount may be determined as a routine matter by one of ordinary skill in the art. In addition, preferably, as used herein, the term "effective content" typically and preferably refers to an amount which is necessary or sufficient to increase release of NO and to inhibit PDE5. The effective amount may vary depending on the particular composition administered and the size of the subject. One of ordinary skill in the art may empirically determine the effective amount of the particular composition of the present invention without the need for undue experimentation.

As used herein, the term "mammal" includes humans, mice, rats, guinea pigs, monkeys, dogs, cats, horses, cattle, pigs and sheep, but is not limited thereto. As used herein, the term "mammal" preferably refers to a human. The term "subject", as used herein, includes, but is not limited to, humans and mammals. The term "subject", as used herein, preferably refers to humans.

The term "pharmaceutically acceptable" refers to properties that do not impair the biological activity and properties of a compound.

As used herein, the term "administration" refers to the act of introducing the active pharmaceutical agents, e.g., PLGA microparticles of the present invention to a subject by any suitable method, e.g. by administration of an array of microneedles as described herein to the skin of the subject and penetration of said microneedles into the skin of the subject, preferably wherein said microparticles reach the dermis of the subject.

As used herein, the term "dermal administration" refers to administration (e.g., administration of the dissolvable microneedles) to the dermis of the skin. In preferred embodiments, dermal administration comprises penetration of the disclosed microneedles through the stratum corneum and delivery of the PLGA microparticles described herein to the dermis.

As used herein the term "% (w/w)" or "(wt/wt)" refers to (mass of the component / total mass of the composition) x 100. In some embodiments, the expression "(wt/wt)" can refer to, e.g., the mass of the compound of Formula (I) in the dissolvable microparticles. In some embodiments, the expression "(wt//wt)" can refer to the mass of the microparticles as a percentage of the mass of the tip layer of the dissolvable microneedles, or to the mass of the microparticles within the total mass of the dissolvable microneedles. Unless otherwise indicated, all percentages herein are given in terms of (wt/wt) percentages.

As used herein the term "% (w/v)" refers to (mass of the component / total volume of the composition) x 100.

As used herein the term "PLGA" is understood as a copolymer of poly lactic acid (PLA) and poly glycolic acid (PGA). PLGA is also referred to herein as poly(lactic-co-glycolic) acid.

As used herein, the term "kit" means that the components comprised in said kit are provided physically separable and distinguishable from one another as different components but are provided or sold together for the purpose of being administered, or used, together.

Accordingly, in one aspect, the present disclosure provides a microneedle, wherein the microneedle comprises a plurality of microparticles, wherein said microparticles comprise poly(lactic-co-glycolic) acid and wherein said microparticles encapsulate a compound Formula (I) or a pharmaceutically acceptable salt thereof;
wherein said compound of Formula (I) has the structure: 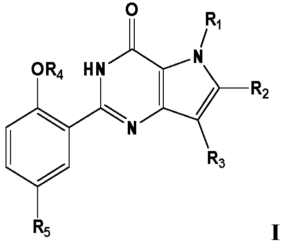 wherein
R₁ is C₁-C₃alkyl, preferably methyl or ethyl, further preferably ethyl;
R₂ is H, CHO or CH=N-OH, preferably H, CHO or (E)-CH=N-OH, further preferably H;
R₃ is C₁-C₄alkyl, preferably ethyl or propyl, further preferably n-propyl;
R₄ is C₁-C₆alkyl, preferably ethyl or propyl, further preferably n-propyl;
R₅ is SO₂NR₆R₇, wherein R₆ and R₇, together with the nitrogen atom to which they are attached, form a heterocyclic ring, wherein said heterocyclic ring is selected from piperidine and piperazine, wherein said heterocyclic ring is substituted with at least one R₈; and
R₈ is independently selected from C₁-C₆alkyl optionally substituted with OH, ONO₂, wherein at least one of said at least one R₈ comprises at least one ONO₂ moiety.

In one aspect, the present invention provides a dissolvable microneedle, wherein the dissolvable microneedle comprises:
(a) a dissolvable polymer; and
(b) a plurality of microparticles, wherein said microparticles comprise poly(lactic-co-glycolic) acid and a compound Formula (I) or a pharmaceutically acceptable salt thereof;
wherein said compound of Formula (I) has the structure: 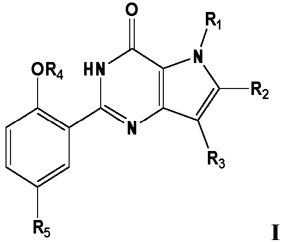 wherein
R₁ is C₁-C₃alkyl, preferably methyl or ethyl, further preferably ethyl;
R₂ is H, CHO or CH=N-OH, preferably H, CHO or (E)-CH=N-OH, further preferably H;
R₃ is C₁-C₄alkyl, preferably ethyl or propyl, further preferably n-propyl;
R₄ is C₁-C₆alkyl, preferably ethyl or propyl, further preferably n-propyl;
R₅ is SO₂NR₆R₇, wherein R₆ and R₇, together with the nitrogen atom to which they are attached, form a heterocyclic ring, wherein said heterocyclic ring is selected from piperidine and piperazine, wherein said heterocyclic ring is substituted with at least one R₈; and
R₈ is independently selected from C₁-C₆alkyl optionally substituted with OH, ONO₂, wherein at least one of said at least one R₈ comprises at least one ONO₂ moiety.

In some embodiments, said dissolvable polymer comprises hyaluronic acid, collagen, polyvinylpyrrolidone, polylactic acid, poly(lactic-co-glycolic) acid, polyglycolic acid, polyethylene glycol, polycaprolactone, poly-amino acid, polypropylene fumarate, poly-gamma glutamic acid, polyvinyl alcohol, polyethyleneimine, albumin, dextran, fibrin, elastin, gelatin, alginic acid, polyacrylic acid, carboxymethylcellulose, and mixtures or copolymers thereof; preferably wherein said dissolvable polymer comprises hyaluronic acid.

In some embodiments, said dissolvable microneedle is a dissolvable candlelit microneedle. In some embodiments, said plurality of microparticles is dispersed within a core layer of said dissolvable candlelit microneedle.

In some embodiments, said core layer of said dissolvable candlelit microneedle comprises said microparticles at a concentration range from about 0.5% (wt/wt) to about 20% (wt/wt).

In some embodiments, said microparticle comprises poly(lactic-co-glycolic) acid, and wherein a ratio of lactic acid:glyclolic acid in said poly(lactic-co-glycolic) acid is from about 15:85 to about 85:15.

In some embodiments, said microparticles comprise said compound of Formula (I) at a concentration from about 0.01% (wt/wt) to about 5% (wt/wt).

In some embodiments, said dissolvable microneedle comprises said PLGA microparticles at a concentration range from about 0.25% (wt/wt) to about 10% (wt/wt).

In some embodiments, said dissolvable microneedle is arrayed on a microneedle supporting surface, wherein preferably said supporting surface is a patch.

In some embodiments, R₁ is methyl or ethyl; R₂ is H; R₃ is ethyl or n-propyl; and R₄ is ethyl or n-propyl.

In some embodiments, said compound of Formula (I) is Compound 1 or Compound 2: 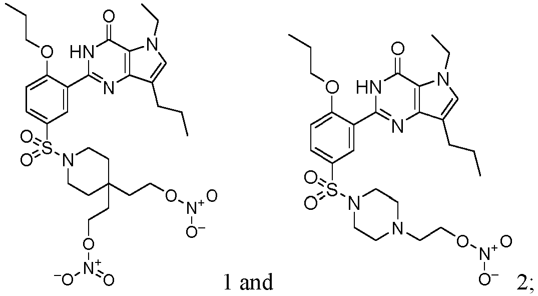 and wherein preferably said compound of Formula (I) is Compound **1;** or a pharmaceutically acceptable salt thereof.

In one aspect, the present disclosure provides a dissolvable microneedle as described herein for use in a method of treating a disease or disorder in a subject in need thereof, preferably wherein said disease or disorder is selected from a skin disease, skin aging, steroid-induced skin atrophy, and erectile dysfunction.

In some embodiments, the dissolvable microneedle is administered to said subject about once every 30 days.

In one aspect, the present disclosure provides a kit comprising:
(i) a microneedle as described herein, preferably wherein said microneedle is arranged in a microneedle array; and
(ii) an applicator for applying said microneedle array to the skin of a subject.

In one aspect, the present disclosure provides a method of preparing a dissolvable microneedle as described herein, the method comprising;
(i) encapsulating a compound of Formula (I) in a microparticle; and
(ii) incorporating said microparticle within said dissolvable microneedle.

In preferred embodiments, said microneedle is a dissolvable candlelit microneedle; said dissolvable polymer is hyaluronic acid; and said compound of Formula (I) is Compound 1.

In one aspect, the present disclosure provides a dissolvable candlelit microneedle, wherein the dissolvable microneedle comprises:
(a) a dissolvable polymer, wherein said dissolvable polymer is hyaluronic acid; and
(b) a plurality of microparticles, wherein said microparticles comprise poly(lactic-co-glycolic) acid and wherein said microparticles encapsulate a compound Formula (I) or a pharmaceutically acceptable salt thereof;
wherein said compound of Formula (I) has the structure:

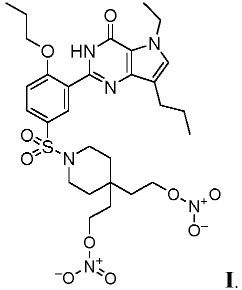

Without wishing to be bound by theory, the present invention combines the advantageous effects of microneedles, preferably dissolvable microneedles and more preferably dissolvable candlelit microneedles, with the sustained release of drugs (e.g., compounds of Formula (I), preferably Compound 1) from PLGA microparticles. One way to enhance patient compliance for the treatment of alopecia in contrast to previous treatments is sustained release of drug over long period of time through the degradation of poly (lactic-co-glycolic acid) (PLGA). PLGA degrades into watersoluble, non-toxic products through hydrolysis and is approved for human use. Also, PLGA itself has been shown to be of benefit for the treatment of alopecia due to the production of lactate resulting from the hydrolysis of PLGA *in vivo.*

The present disclosure teaches a drug delivery method utilizing PLGA microparticles dispersed within microneedles, preferably dissolvable microneedles and more preferably dissolvable candlelit microneedles (CMNs) to ensure effective and patient-compliant treatment of skin disorders such as alopecia. In preferred embodiments, the PLGA microparticles comprise (preferably, encapsulate) a compound of Formula (I), preferably Compound 1, for the treatment of a disease or disorder, e.g., to promote hair growth. Compound 1 is a dual-pharmacology agent capable of releasing NO and inhibiting PDE5 *in vivo.* By encapsulating compounds of Formula (I) into PLGA microparticles, sustained release of the compounds of Formula (I) was achieved, significantly reducing the number of administrations needed to promote hair growth and, in preferred embodiments, increasing drug efficacy and patient compliance.

As shown in the Examples herein, a single application of the dissolvable candlelit microneedles containing PLGA-microparticles (preferably PLGA-microparticles encapsulating a compound of Formula (I), preferably Compound 1) showed similar hair growth effects as 14 applications of topical minoxidil over a 28 day period.

The present disclosure relates to microneedles, preferably dissolvable microneedles, and most preferably dissolvable candlelit microneedles, wherein the microneedles comprise a plurality of PLGA microparticles, wherein the microparticles comprise a compound Formula (I) or a pharmaceutically acceptable salt thereof.

### Dissolvable Microneedles

Microneedles (MNs) are a type of intradermal drug delivery system that provide a minimally invasive method of drug delivery into the skin, preferably the dermis, and preferably by bypassing the stratum corneum layer of the skin.

Without wising to be bound by theory, microneedles can create a micrometer (µm)-sized hole in the skin surface of a subject after inoculation of the subject with the microneedle. In preferred embodiments, the microneedle is a dissolvable microneedle comprising PLGA microparticles. When the microneedle is administered to the skin of a subject, the microneedle dissolves (e.g., within about 60 minutes, e.g., within about 30 minutes, preferably within about 15 minutes), leaving behind the microparticles that were previously dispersed therein in the hole made by the microneedle penetration and thus leading to absorption of the microparticles into the skin. When one or more microneedles (preferably dissolvable microneedles) as described herein are arrayed on a support structure such as a patch, the result is that the microparticles are applied to the skin of a subject over the whole area of the support structure (e.g., patch).

Various types of microneedles can be used in accordance with the present disclosure, including in-plane type microneedles (Lin Liwei, et al., "Silicon-processed Microneedles", Journal of Microelectromechanical Systems: a joint IEEE and ASME publication on microstructures, microactuators, microsensors, and microsystems 8(1): 78-84(1999)); biodegradable polymer microneedles (*e.g.,* Jung-Hwan Park, et al., "Biodegradable polymer microneedles: Fabrication, mechanics and transdermal drug delivery", Journal of Controlled Release 104 (1):51-66 (2005)); absorbable microneedles (*e.g*., Japanese Patent Application Laid-Open No. 2005154321; and Takaya Miyano, et al., "Sugar Micro Needles as Transdermic Drug Delivery System", Biomedical Microdevices, 7(3):185-188(2005)); biodegradable microneedles (*e.g.,* Sean P Sullivan, et al., "Minimally Invasive Protein Delivery with Rapidly Dissolving Polymer Microneedles", Advanced Materials 20(5):933- 938 (2008)); hollow microneedles (*e.g*., Korean Patent Laid-Open No. 10-2016-0024354); and biodegradable polymer microneedles suitable for quantitative delivery of a drug (*e.g*., Korean Patent Laid-Open No. 10-2020-0024515). In addition, as a form for injecting the compound or pharmaceutical composition, microneedles manufactured using a centrifugal force (*e.g.,* PCT Patent No. PCT/KR2014/003964) may be used.

In preferred embodiments, the microneedles of the present disclosure are dissolvable microneedles. The terms "dissolvable microneedle" and "dissolving microneedle" are used interchangeably herein. In preferred embodiments, when a plurality of microneedles are used, the microneedles are dissolvable microneedles.

In preferred embodiments, the dissolvable microneedles of the present disclosure comprise (e.g., are made of) a dissolvable polymer, preferably not including, i.e., exclusive of, the PLGA microparticles dispersed therein. As used herein, a "dissolvable polymer" is understood to mean a polymer that dissolves, i.e., degrades into its constituent monomers, when applied intradermally to skin (e.g., after penetration of the stratum corneum). In preferred embodiments, the dissolvable polymer (and thus the dissolvable microneedle) dissolves within at least 24 hours when applied intradermally to skin (e.g., after penetration of the stratum corneum). Preferably, said dissolvable polymers (and thus the dissolvable microneedles) dissolve within 12 hours, preferably within 6 hours, more preferably within 1 hour, yet more preferably within 30 minutes, yet more preferably within 15 minutes when applied intradermally to skin (e.g., after penetration of the stratum corneum).

As described in the Examples, in a preferred embodiment, the dissolvable polymer of the dissolvable microneedles is hyaluronic acid and said dissolvable microneedles dissolve within about 15 minutes when applied intradermally to skin (e.g., after penetration of the stratum corneum). Thus, in preferred embodiments, the dissolvable polymers (and thus the dissolvable microneedles) dissolve within a time range from about 5 minutes to about 25 minutes, preferably within a time range from about 10 minutes to about 20 minutes when applied intradermally to skin. Without wishing to be bound by theory, hyaluronic acid (HA) is a glycosaminoglycan comprising repeating disaccharide units. When HA is administered to a subject, the glycosaminoglycan polymer is hydrolyzed to its constituent disaccharide units.

In preferred embodiments, when said dissolvable polymer (preferably HA) of said dissolvable microneedle dissolves upon application intradermally to human skin, the dissolution of said dissolvable polymer enables dermal delivery of the PLGA microparticles previously dispersed within the dissolvable microneedles into the dermis of a subject. In preferred embodiments, the dissolvable microneedles of the present disclosure comprise PLGA microparticles, wherein said PLGA microparticles are released into the dermis upon dissolution of the dissolvable microneedles, preferably wherein said PLGA microparticles comprise a compound of Formula (I), preferably Compound 1.

In some embodiments, at least 80% of said dissolvable microneedle (preferably exclusive of said PLGA microparticles) is a dissolvable polymer, preferably hyaluronic acid. In some embodiments, at least 90% of said dissolvable microneedle (preferably exclusive of said PLGA microparticles) is a dissolvable polymer, preferably hyaluronic acid. In some embodiments, at least 95% of said dissolvable microneedle (preferably exclusive of said PLGA microparticles) is a dissolvable polymer, preferably hyaluronic acid. In some embodiments, at least 99% of said dissolvable microneedle (preferably exclusive of said PLGA microparticles) is a dissolvable polymer, preferably hyaluronic acid.

In preferred embodiments, the dissolvable microneedle consists essentially of a dissolvable polymer (preferably wherein said consisting essentially of does not include said PLGA microparticles). In preferred embodiments, the dissolvable microneedle consists of a dissolvable polymer (preferably wherein said consisting of does not include said PLGA microparticles).

In preferred embodiments, the dissolvable polymer is a biocompatible dissolvable polymer. In preferred embodiments, the dissolvable microneedles consists of or consists essentially of the biocompatible dissolvable polymer. As used herein, the term "biocompatible dissolvable polymer" refers to a material that is not considered substantially toxic to the human body, is preferably chemically inert and preferably does not exhibit immunogenicity. In preferred embodiments, the biocompatible dissolvable polymer can be degraded by body fluids or microorganisms *in vivo.* In preferred embodiments, the biocompatible dissolvable polymer is generally regarded as safe (GRAS) under sections 201(s) and 409 of the United States Federal Food, Drug, and Cosmetic Act.

In preferred embodiments, the dissolvable polymer (e.g., the biocompatible dissolvable polymer) comprises hyaluronic acid, collagen, polyvinylpyrrolidone, polylactic acid, poly(lactic-co-glycolic) acid, polyglycolic acid, polyethylene glycol, polycaprolactone, poly-amino acid, polypropylene fumarate, poly-gamma glutamic acid, polyvinyl alcohol, polyethyleneimine, albumin, dextran, fibrin, elastin, gelatin, alginic acid, polyacrylic acid, carboxymethylcellulose, chitosan, silk fibroin, and mixtures or copolymers thereof.

In preferred embodiments, said dissolvable polymer (e.g., the biocompatible dissolvable polymer) comprises hyaluronic acid or poly(lactic-co-glycolic) acid; or mixtures or copolymers thereof. In some embodiments, said dissolvable polymer (e.g., the biocompatible dissolvable polymer) comprises poly(lactic-co-glycolic) acid.

In preferred embodiments, said dissolvable polymer (e.g., the biocompatible dissolvable polymer) comprises carboxymethylcellulose. In preferred embodiments, said dissolvable polymer (e.g., the biocompatible dissolvable polymer) comprises polyvinylpyrrolidone. In preferred embodiments, said dissolvable polymer (e.g., the biocompatible dissolvable polymer) comprises polyvinyl alcohol. In preferred embodiments, said dissolvable polymer (e.g., the biocompatible dissolvable polymer) comprises polyacrylic acid. In preferred embodiments, said dissolvable polymer (e.g., the biocompatible dissolvable polymer) comprises polylactic acid. In preferred embodiments, said dissolvable polymer (e.g., the biocompatible dissolvable polymer) comprises chitosan. In preferred embodiments, said dissolvable polymer (e.g., the biocompatible dissolvable polymer) comprises silk fibroin.

In preferred embodiments, said dissolvable polymer (e.g., the biocompatible dissolvable polymer) comprises hyaluronic acid. In some embodiments, said dissolvable polymer (e.g., the biocompatible dissolvable polymer) comprises at least 80% hyaluronic acid. In preferred embodiments, said dissolvable polymer (e.g., the biocompatible dissolvable polymer) comprises at least 90% hyaluronic acid. In preferred embodiments, said dissolvable polymer (e.g., the biocompatible dissolvable polymer) comprises at least 95% hyaluronic acid. In preferred embodiments, said dissolvable polymer (e.g., the biocompatible dissolvable polymer) comprises at least 99% hyaluronic acid. In preferred embodiments, said dissolvable polymer (e.g., the biocompatible dissolvable polymer) consists essentially of hyaluronic acid. In preferred embodiments, said dissolvable polymer (e.g., the biocompatible dissolvable polymer) consists of hyaluronic acid.

In preferred embodiments, the microneedles of the present disclosure can penetrate the skin of a subject, preferably to a depth from between about 500 µm to about 1000 µm, preferably from about 600 µm to about 900 µm, more preferably to a depth of about 750 µm (e.g., after penetration of the stratum corneum).

### Candlelit Microneedles

The most preferred type of microneedles are candlelit microneedles, most preferably wherein said candlelit microneedles are dissolvable candlelit microneedles. Candlelit microneedles, also abbreviated as "CMNs") are described in, e.g., WO 2020/075997 (see US 2021/0393933 and EP 3 848 085 for English-language translations thereof). Accordingly, in preferred embodiments, the microneedle is a candlelit microneedle. In yet more preferred embodiments, the microneedle is a dissolvable candlelit microneedle.

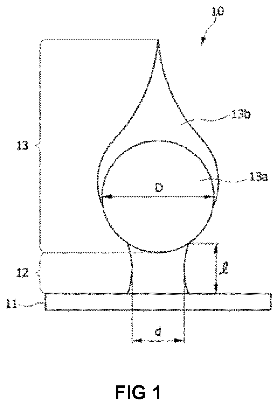

A preferred candlelit-shaped microneedle according to the present invention is shown in FIG 1. As shown in FIG 1, in preferred embodiments, the candlelit microneedle comprises a tip layer having a candle-shaped cross section.

In preferred embodiments, the candlelit microneedle and includes:
(i) an insertion layer (13), preferably wherein said insertion layer comprises:
   (a) a core layer (13a); and
   (b) a tip layer (13b);
(ii) a base layer (12); wherein said base layer is attached to
(iii) a substrate (11).

In preferred embodiments, said base layer has a height "*l*" and a diameter "d". In preferred embodiments, said core layer has a diameter "D".

In preferred embodiments, said candlelit microneedle further comprises a separation layer formed between said substrate (11) and said base layer (12). In preferred embodiments, said separation layer physically separates the base layer (12) from the substrate (11) upon the application of an external force, e.g., a pulling force pulling the substrate and the base layer in different directions. For example, in preferred embodiments, after a candlelit microneedle is inserted into the skin (preferably the dermis) of a subject, said substrate is pulled back away from the skin of said subject. The pulling back of the substrate causes the separation layer to separate, leaving the candlelit microneedle(s) in the skin (preferably the dermis) of the subject while the substrate is removed. In preferred embodiments, the external force necessary to destroy the separation layer and thereby separate the base layer from the substrate is less than the force necessary to remove the inserted candlelit microneedle(s) from the skin of the subject.

Accordingly, in preferred embodiments, the dissolvable candlelit microneedles are inserted into the skin of a subject, and the insertion layer, (i.e., the tip layer and the core layer) and the base layer of the dissolvable candlelit microneedles are separated from the substrate via the separation layer. The result is that the insertion layer (i.e., the tip layer and the core layer) and the base layer remain in the skin of the subject, while the substrate does not penetrate the skin of the subject and is removed from the skin of the subject.

In preferred embodiments, the separation layer may be broken by a force smaller than the force necessary to remove the insertion and/or base layer from the skin.

In preferred embodiments, the external force may be applied in a horizontal direction or a vertical direction with respect to the separation layer.

In preferred embodiments, the separation layer or the base layer may include a biodegradable material, preferably wherein said biodegradable material is carboxymethyl cellulose, hyaluronic acid, polyvinylpyrrolidone, or a mixture thereof.

In preferred embodiments, a solvent can be used to weaken the separation layer and thereby reduce the amount of external force necessary to destroy the separation layer and thus separate the substrate from the base layer. In preferred embodiments, the separation can be dissolved by the solvent. In some embodiments, the time required for the separation layer to be dissolved by the solvent is less than 5 minutes. In preferred embodiments, 70% or more of the separation layer per unit volume is dissolved by the solvent within 3 minutes. In some embodiments, the solvent is introduced between the substrate and the skin or is applied on the skin before the tip portion is inserted into the skin. In some embodiments, said solvent comprises water, an alcohol comprising from one to four carbon atoms, acetone, ethyl acetate, chloroform, 1,3-butylene glycol, diethyl ether, butyl acetate, propylene glycol, polyethylene glycol, ethyl oleate, or an oil (e.g., vegetable oil, mineral oil, silicone oil or synthetic oil).

In preferred embodiments, the base layer serves as a supporting structure to help enable the core layer and tip layer to be completely inserted into the skin.

In preferred embodiments, the cross-section of a central portion of the base layer has a smaller area than the cross-sections of both sides of the base layer as shown in FIG 1.

In preferred embodiments, the substrate may be formed with a protrusion having a predetermined thickness in a portion corresponding to the separation layer formed on one surface thereof.

In preferred embodiments, the protrusion may be formed in a horizontal straight line, an inclined oblique line, or a convex or concave curve in the cross section of the surface on which the separation layer is formed.

### Methods of Preparing the Candlelit Microneedles

In one aspect, the present disclosure provides a method of preparing a dissolvable microneedle as described herein, the method comprising;
(i) preparing a PLGA microparticle; and
(ii) incorporating said microparticle within said dissolvable microneedle.

In some embodiments, incorporating said microparticle within said dissolvable microneedle comprises:
(a) preparing a base layer of said microneedle, preferably wherein preparing said base layer of said microneedle comprises depositing droplets of an aqueous solution of a dissolvable polymer, preferably hyaluronic acid on a substrate, covering said droplets with a plate cover wherein said plate cover is in contact with said droplets, and allowing said droplets of aqueous solution to dry;
(b) preparing a core layer of said microneedle, preferably wherein preparing said core layer of said microneedle comprises depositing an aqueous solution comprising said dissolvable polymer, preferably hyaluronic acid and said microparticles on said base layer and allowing said aqueous solution to dry;
(c) preparing a tip layer of said microneedle, preferably wherein preparing said tip layer of said microneedle comprises depositing an aqueous solution of said dissolvable polymer, preferably hyaluronic acid on said core layer, preferably further comprising subjecting said microneedle to centrifugal lithography.

In preferred embodiments, said preparing a PLGA microparticle comprises mixing PLGA in an aqueous solution, and allowing the mixture to dry.

In preferred embodiments, said preparing a core layer comprises pre-mixing said PLGA microparticles with said aqueous solution of said dissolvable polymer, preferably hyaluronic acid prior to dispensing said aqueous solution of said dissolvable polymer on said base layer and allowing said aqueous solution to dry.

### Supporting Surfaces

In preferred embodiments, the microneedles, preferably the dissolvable microneedles and more preferably the dissolvable candlelit microneedles, are mounted on a supporting surface. The supporting surface can be arranged on top of the substrate (i.e., Item 11 in FIG 1), and can be used to keep a microneedle array intact after removal of the array from the substrate. In preferred embodiments, the supporting surface can facilitate administration of a microneedle array to the skin of a subject.

The supporting surface may be fabricated from a variety of materials, including but not limited to polyurethane film, plastic, fabric or metal. When the supporting surface is made from plastic, said supporting surface can be made by injection molding. In preferred embodiments, the supporting surface exhibits mechanical strength, flexibility, and compatibility with the microneedle formulation. In preferred embodiments, said supporting surface is a polyurethane patch, preferably wherein said patch further comprises an array of microneedles. In some embodiments, said supporting surface is a medical fabric (e.g., a band of medical fabric), preferably wherein said medical fabric comprises an array of microneedles.

In preferred embodiments, the microneedle of the present invention is arrayed on the support structure. As used herein, the term "array" refers to an organized configuration in which multiple components, preferably microneedles, are systemically positioned in a specific geometric pattern. The components can be arranged in, e.g., a circular, rectangular or other defined formation, preferably wherein each component spaced at uniform intervals (e.g., wherein said intervals are from about 1.0 mm to about 1.5 mm). Said arrays can be dispensed, e.g., using an automated, programmed dispenser. In preferred embodiments, arrays are used to maximize coverage of the microneedles over a particular area. In preferred embodiments, arrays enable uniform administration of the microneedles and microparticles disclosed herein, leading to enhanced performance of the microparticle-containing microneedles.

The size and shape of the supporting surface (e.g., patch), preferably comprising a microneedle array, can be adjusted to cover skin areas of different sizes and shapes. In some embodiments, the supporting surfaces (e.g., patches) vary from small, localized arrays (e.g., patches) to larger arrays (e.g., patches) suitable for extensive treatment areas. Without wishing to be bound by theory, the flexibility of design enables the supporting surface (e.g., patch) to conform to different anatomical surfaces, thereby enhancing the contact between the microneedles and the skin and preferably increasing the penetration of the microneedles into the skin.

In some embodiments, the supporting surface (e.g., patch) includes a backing layer or structural reinforcement, e.g., to maintain the integrity of the patch during application and storage. In some embodiments, said patch is an adhesive patch. In some embodiments, said patch is a non-adhesive patch.

A "microneedle applicator" as described herein comprises a rigid surface capable of securely and uniformly attaching the microneedle supporting surface (e.g., patch; preferably comprising an array of microneedles) during application. The rigid surface ensures that the microneedles (preferably the arrayed microneedles) maintain their alignment and positioning relative to the skin or target area. The microneedle applicator preferably includes a mechanism for generating force, such as a spring, which provides the necessary thrust to drive the microneedles through the stratum corneum and into the dermis. Any component capable of generating sufficient thrust force to facilitate microneedle penetration may be employed. In preferred embodiments, the microneedle applicator is equipped with a user-operative device, such as a handle, trigger, or button, that allows for precise control over the initiation of the thrust force. This feature enables the user to determine the exact moment when the application process begins, ensuring accurate and consistent delivery of the microneedles into the skin. In preferred embodiments, the design of the applicator includes ergonomic considerations to enhance user comfort and ease of use, and safety mechanisms to prevent accidents.

In preferred embodiments, the microneedle of the present invention is mounted on flexible or rigid support structure, upon which microneedles, preferably dissolvable microneedles, are arrayed.

In preferred embodiments, the microneedle patch can be designed to be either adhesive or non-adhesive.

In preferred embodiments, the microneedle support structure, e.g., patch size can vary. In preferred embodiments, the microneedle support structure has a size from about 10 mm² to about 1000 mm², preferably from about 45 mm² to about 750 mm².

In preferred embodiments, the microneedle support structure, e.g., patch shape can vary.

In preferred embodiments, microneedle of the present invention is used with microneedle applicator, comprised of rigid surface to support the microneedle during application.

In preferred embodiments, the microneedle applicator includes a force-generating mechanism, such as spring, but not limited to spring. In preferred embodiments, the microneedle applicator includes a user-operative device, such as a handle, trigger, or button, but not limited thereto. In preferred embodiments, the microneedle applicator includes ergonomic design considerations to enhance user comfort and ease of use.

In one aspect, the present disclosure provides a kit comprising: (i) a microneedle as described herein, preferably wherein said microneedle is arranged in a microneedle array; and (ii) an applicator for applying said microneedle array to the skin of a subject.

In some embodiments, said microneedle array is arranged on an adhesive or non-adhesive patch.

In preferred embodiments, said microneedle array is arranged on an adhesive patch, wherein said adhesive patch can be applied to the skin of a subject, preferably a human.

### PLGA Microparticles

In very preferred embodiments, the microneedles are dissolvable microneedles, wherein said dissolvable microneedles comprise a dissolvable polymer, and wherein said dissolvable microneedles further comprise a PLGA microparticle (MP) comprising a compound of Formula (I). As used herein, the abbreviation "MP" refers to "PLGA microparticle", and the abbreviation "MP-CMN" refers to PLGA microparticle-comprising candlelit microneedles.

In preferred embodiments, the PLGA microparticles are dispersed within the microneedles (preferably dissolvable candlelit microneedles) described herein. The term "dispersed within" or "dispersed therein" is understood to mean that the PLGA microparticles are incorporated within the dissolvable polymer (e.g., hyaluronic acid) that the microneedles are made of, preferably wherein said PLGA microparticles are homogenously (i.e., substantially uniformly) incorporated within the dissolvable polymer (e.g., hyaluronic acid) that makes up the microneedle. As described in the Examples below, microparticle-containing microneedles are preferably prepared by mixing the PLGA microparticles in a solution comprising the dissolvable polymer of the microneedles (preferably HA) to prepare a homogenous dispersion of PLGA microparticles in the dissolvable polymer. When the solvent of the solution (e.g., water or PBS) comprising the dissolvable polymer and PLGA microparticles is removed (e.g., by drying), the result is a polymer matrix (e.g., an HA matrix) comprising the PLGA microparticles dispersed therein. In preferred embodiments, the PLGA microparticles are uniformly distributed within at least a portion of the microneedles (e.g., the PLGA microparticles are uniformly dispersed within the insertion layer, preferably the core layer of the microneedles). In some embodiments, the microparticles comprise poly(lactic-co-glycolic) acid, wherein the ratio of lactic acid:glycolic acid in said poly(lactic-co-glycolic) acid is from about 15:85 to about 85:15.

In some embodiments, the microparticles comprise poly(lactic-co-glycolic) acid, wherein the ratio of lactic acid:glycolic acid in said poly(lactic-co-glycolic) acid is from about 25:75 to about 85:15. In some embodiments, the microparticles comprise poly(lactic-co-glycolic) acid, wherein the ratio of lactic acid:glycolic acid in said poly(lactic-co-glycolic) acid is from about 35:65 to about 85:15. In some embodiments, the microparticles comprise poly(lactic-co-glycolic) acid, wherein the ratio of lactic acid:glycolic acid in said poly(lactic-co-glycolic) acid is from about 50:50 to about 85:15. In some embodiments, the microparticles comprise poly(lactic-co-glycolic) acid, wherein the ratio of lactic acid:glycolic acid in said poly(lactic-co-glycolic) acid is from about 60:40 to about 85:15. In some embodiments, the microparticles comprise poly(lactic-co-glycolic) acid, wherein the ratio of lactic acid:glycolic acid in said poly(lactic-co-glycolic) acid is from about 60:40 to about 80:20. In some embodiments, the microparticles comprise poly(lactic-co-glycolic) acid, wherein the ratio of lactic acid:glycolic acid in said poly(lactic-co-glycolic) acid is from about 60:40 to about 75:25. In some embodiments, the microparticles comprise poly(lactic-co-glycolic) acid, wherein the ratio of lactic acid:glycolic acid in said poly(lactic-co-glycolic) acid is from about 60:40 to about 70:30. In some embodiments, the microparticles comprise poly(lactic-co-glycolic) acid, wherein the ratio of lactic acid:glycolic acid in said poly(lactic-co-glycolic) acid is about 25:75. In some embodiments, the microparticles comprise poly(lactic-co-glycolic) acid, wherein the ratio of lactic acid:glycolic acid in said poly(lactic-co-glycolic) acid is about 50:50. In some embodiments, the microparticles comprise poly(lactic-co-glycolic) acid, wherein the ratio of lactic acid:glycolic acid in said poly(lactic-co-glycolic) acid is about 60:40. In some embodiments, the microparticles comprise poly(lactic-co-glycolic) acid, wherein the ratio of lactic acid:glycolic acid in said poly(lactic-co-glycolic) acid is about 65:35. In some embodiments, the microparticles comprise poly(lactic-co-glycolic) acid, wherein the ratio of lactic acid:glycolic acid in said poly(lactic-co-glycolic) acid is about 70:30. In some embodiments, the microparticles comprise poly(lactic-co-glycolic) acid, wherein the ratio of lactic acid:glycolic acid in said poly(lactic-co-glycolic) acid is about 75:25.

In preferred embodiments, said PLGA microparticles are dispersed within the core layer of said candlelit microneedles. In preferred embodiments, the tip layer of said candlelit microneedle does not comprise said PLGA microparticles.

In preferred embodiments, said base layer of said microneedle does not comprise microparticles.

In preferred embodiments, said separation layer of said microneedle does not comprise microparticles.

In preferred embodiments, the PLGA microparticles have an average size (diameter) from about 0.1µm to about 100 µm. In more preferred embodiments, the PLGA microparticles have an average size (diameter) from about 1.0 µm to about 50 µm.

In preferred embodiments, said PLGA microparticles dissolve (i.e., the PLGA polymer of the microparticles breaks down into lactate and glycolate) after administration of the microparticles to the skin (preferably the dermis) of a subject. In preferred embodiments, the microparticles dissolve within a period from about one to about ten weeks, preferably from about two to about eight weeks, more preferably from about three to about five weeks. In preferred embodiments, said dissolution of said microparticles releases the compounds of Formula (I).

### Compounds of Formula (I)

In preferred embodiments, the PLGA microparticles comprise a compound of Formula (I). As described in the Examples, the compound of Formula (I)-containing microparticles are formed by combining PLGA and a compound of Formula (I) in solution and subsequent removal of the solvent. Accordingly, in preferred embodiments, compounds of Formula (I) are homogenously dispersed within the PLGA microparticles. As used herein, PLGA microparticles wherein a compound of Formula (I) is homogenously dispersed therein can be said to "contain" or to "encapsulate" said compound of Formula (I).

In some embodiments, the PLGA microparticles comprise said compound of Formula (I), preferably Compound 1, at a concentration from about 0.01% (w/w) to about 5% (w/w). In some embodiments, the PLGA microparticles comprise said compound of Formula (I), preferably Compound 1 at a concentration from about 0.05% (w/w) to about 5% (w/w). In some embodiments, the PLGA microparticles comprise said compound of Formula (I), preferably Compound 1, at a concentration from about 0.1% (w/w) to about 5% (w/w). In some embodiments, the PLGA microparticles comprise said compound of Formula (I), preferably Compound 1, at a concentration from about 0.1% (w/w) to about 2.5% (w/w). In some embodiments, the PLGA microparticles comprise said compound of Formula (I), preferably Compound 1, at a concentration from about 0.1% (w/w) to about 1% (w/w). In some embodiments, the PLGA microparticles comprise said compound of Formula (I), preferably Compound 1, at a concentration from about 0.1% (w/w) to about 0.9% (w/w). In some embodiments, the PLGA microparticles comprise said compound of Formula (I), preferably Compound 1, at a concentration from about 0.25% (w/w) to about 0.75% (w/w).

In some embodiments, the PLGA microparticles comprise about 0.1% (w/w) of a compound of Formula (I), preferably Compound 1. In some embodiments, the PLGA microparticles comprise about 0.2% (w/w) of a compound of Formula (I), preferably Compound 1. In some embodiments, the PLGA microparticles comprise about 0.3% (w/w) of a compound of Formula (I), preferably Compound 1. In some embodiments, the PLGA microparticles comprise about 0.4% (w/w) of a compound of Formula (I), preferably Compound 1. In some embodiments, the PLGA microparticles comprise about 0.5% (w/w) of a compound of Formula (I), preferably Compound 1. In some embodiments, the PLGA microparticles comprise about 0.6% (w/w) of a compound of Formula (I), preferably Compound 1. In some embodiments, the PLGA microparticles comprise about 0.7% (w/w) of a compound of Formula (I), preferably Compound 1. In some embodiments, the PLGA microparticles comprise about 0.8% (w/w) of a compound of Formula (I), preferably Compound 1. In some embodiments, the PLGA microparticles comprise about 0.9% (w/w) of a compound of Formula (I), preferably Compound 1. In some embodiments, the PLGA microparticles comprise about 1.0% (w/w) of a compound of Formula (I), preferably Compound 1.

In some embodiments, said core layer of said microneedle, preferably said dissolvable candlelit microneedle, comprises said PLGA microparticles at a concentration range from about 0.5% (wt/wt) to about 20% (wt/wt). In some embodiments, said core layer of said microneedle, preferably said dissolvable candlelit microneedle, comprises said PLGA microparticles at a concentration range from about 0.5% (wt/wt) to about 10% (wt/wt). In some embodiments, said core layer of said microneedle, preferably said dissolvable candlelit microneedle, comprises said PLGA microparticles at a concentration range from about 0.75% (wt/wt) to about 10% (wt/wt). In some embodiments, said core layer of said microneedle, preferably said dissolvable candlelit microneedle, comprises said PLGA microparticles at a concentration range from about 1% (wt/wt) to about 10% (wt/wt). In some embodiments, said core layer of said microneedle, preferably said dissolvable candlelit microneedle, comprises said PLGA microparticles at a concentration range from about 2.5% (wt/wt) to about 7.5% (wt/wt). In some embodiments, said core layer of said microneedle, preferably said dissolvable candlelit microneedle, comprises said PLGA microparticles at a concentration range from about 3% (wt/wt) to about 6% (wt/wt).

In some embodiments, said core layer of said microneedle, preferably said dissolvable candlelit microneedle, comprises about 1% (wt/wt) of said microparticles. In some embodiments, said core layer of said microneedle, preferably said dissolvable candlelit microneedle, comprises about 2% (wt/wt) of said microparticles. In some embodiments, said core layer of said microneedle, preferably said dissolvable candlelit microneedle, comprises about 3% (wt/wt) of said microparticles. In some embodiments, said core layer of said microneedle, preferably said dissolvable candlelit microneedle, comprises about 4% (wt/wt) of said microparticles. In some embodiments, said core layer of said microneedle, preferably said dissolvable candlelit microneedle, comprises about 5% (wt/wt) of said microparticles. In some embodiments, said core layer of said microneedle, preferably said dissolvable candlelit microneedle, comprises about 6% (wt/wt) of said microparticles. In some embodiments, said core layer of said microneedle, preferably said dissolvable candlelit microneedle, comprises about 7% (wt/wt) of said microparticles. In some embodiments, said core layer of said microneedle, preferably said dissolvable candlelit microneedle, comprises about 8% (wt/wt) of said microparticles. In some embodiments, said core layer of said microneedle, preferably said dissolvable candlelit microneedle, comprises about 9% (wt/wt) of said microparticles. In some embodiments, said core layer of said microneedle, preferably said dissolvable candlelit microneedle, comprises about 10% (wt/wt) of said microparticles.

In some embodiments, said core layer of said microneedle, preferably said dissolvable candlelit microneedle, comprises said PLGA microparticles at a concentration range from about 0.5% (wt/wt) to about 20% (wt/wt), and the PLGA microparticles comprise a compound of Formula (I), preferably Compound 1 at a concentration range from about 0.01% (w/w) to about 5% (w/w). In some embodiments, said core layer of said microneedle, preferably said dissolvable candlelit microneedle, comprises said PLGA microparticles at a concentration range from about 0.5% (wt/wt) to about 10% (wt/wt), and the PLGA microparticles comprise a compound of Formula (I), preferably Compound 1 at a concentration range from about 0.05% (w/w) to about 5% (w/w) 1. In some embodiments, said core layer of said microneedle, preferably said dissolvable candlelit microneedle, comprises said PLGA microparticles at a concentration range from about 0.75% (wt/wt) to about 10% (wt/wt), and the PLGA microparticles comprise a compound of Formula (I), preferably Compound 1 at a concentration range from about 0.1% (w/w) to about 5% (w/w). In some embodiments, said core layer of said microneedle, preferably said dissolvable candlelit microneedle, comprises said PLGA microparticles at a concentration range from about 1% (wt/wt) to about 10% (wt/wt), and the PLGA microparticles comprise a compound of Formula (I), preferably Compound 1 at a concentration range from about 0.1% (w/w) to about 2.5% (w/w). In some embodiments, said core layer of said microneedle, preferably said dissolvable candlelit microneedle, comprises said PLGA microparticles at a concentration range from about 2.5% (wt/wt) to about 7.5% (wt/wt), and the PLGA microparticles comprise a compound of Formula (I), preferably Compound 1 at a concentration range from about 0.1% (w/w) to about 1% (w/w). In some embodiments, said core layer of said microneedle, preferably said dissolvable candlelit microneedle, comprises said PLGA microparticles at a concentration range from about 3% (wt/wt) to about 6% (wt/wt), and the PLGA microparticles comprise a compound of Formula (I), preferably Compound 1 at a concentration range from about 0.1% (w/w) to about 0.9% (w/w). In some embodiments, said core layer of said microneedle, preferably said dissolvable candlelit microneedle, comprises said PLGA microparticles at a concentration range from about 3% (wt/wt) to about 6% (wt/wt), and the PLGA microparticles comprise a compound of Formula (I), preferably Compound 1 at a concentration range from about 0.25% (w/w) to about 0.75% (w/w).

In some embodiments, said microneedle, preferably said dissolvable candlelit microneedle, comprises said microparticles at a concentration range from about 0.25% (wt/wt) to about 10% (wt/wt). In some embodiments, said microneedle, preferably said dissolvable candlelit microneedle, comprises said microparticles at a concentration range from about 0.5% (wt/wt) to about 10% (wt/wt). In some embodiments, said microneedle, preferably said dissolvable candlelit microneedle, comprises said microparticles at a concentration range from about 1% (wt/wt) to about 10% (wt/wt). In some embodiments, said microneedle, preferably said dissolvable candlelit microneedle, comprises said microparticles at a concentration range from about 1% (wt/wt) to about 7.5% (wt/wt). In some embodiments, said microneedle, preferably said dissolvable candlelit microneedle, comprises said microparticles at a concentration range from about 1% (wt/wt) to about 5% (wt/wt). In some embodiments, said microneedle, preferably said dissolvable candlelit microneedle, comprises said microparticles at a concentration range from about 1.5% (wt/wt) to about 4% (wt/wt). In some embodiments, said microneedle, preferably said dissolvable candlelit microneedle, comprises said microparticles at a concentration range from about 2% (wt/wt) to about 3% (wt/wt).

In some embodiments, said core layer of said microneedle, preferably said dissolvable candlelit microneedle, comprises compound of Formula I, preferably said Compound 1, at a concentration range from about 0.0025% (wt/wt) to about 0.25% (wt/wt).

In some embodiments, said core layer of said microneedle, preferably said dissolvable candlelit microneedle, comprises compound of Formula I, preferably said Compound 1, at a concentration range from about 0.005% (wt/wt) to about 0.1% (wt/wt).

In some embodiments, said core layer of said microneedle, preferably said dissolvable candlelit microneedle, comprises compound of Formula I, preferably said Compound 1, at a concentration range from about 0.01% (wt/wt) to about 0.1% (wt/wt).

In some embodiments, said core layer of said microneedle, preferably said dissolvable candlelit microneedle, comprises compound of Formula I, preferably said Compound 1, at a concentration range from about 0.01% (wt/wt) to about 0.05% (wt/wt).

In some embodiments, said microneedle, preferably said dissolvable candlelit microneedle, comprises said compound of Formula I, preferably said Compound 1, at a concentration range from about 0.001% (wt/wt) to about 0.1% (wt/wt).

In some embodiments, said microneedle, preferably said dissolvable candlelit microneedle, comprises said compound of Formula I, preferably said Compound 1, at a concentration range from about 0.001% (wt/wt) to about 0.05% (wt/wt).

In some embodiments, said microneedle, preferably said dissolvable candlelit microneedle, comprises said compound of Formula I, preferably said Compound 1, at a concentration range from about 0.005% (wt/wt) to about 0.1% (wt/wt).

In some embodiments, said microneedle, preferably said dissolvable candlelit microneedle, comprises said compound of Formula I, preferably said Compound 1, at a concentration range from about 0.005% (wt/wt) to about 0.05% (wt/wt).

In preferred embodiments, said compound of Formula (I) has the formula: 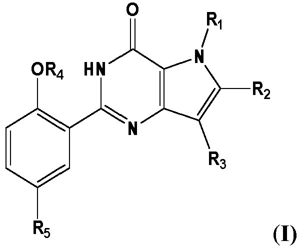 wherein
R₁ is C₁-C₃alkyl, preferably methyl or ethyl, further preferably ethyl;
R₂ is H, CHO or CH=N-OH, preferably H, CHO or (E)-CH=N-OH, further preferably H;
R₃ is C₁-C₄alkyl, preferably ethyl or propyl, further preferably n-propyl;
R₄ is C₁-C₆alkyl, preferably ethyl or propyl, further preferably n-propyl;
R₅ is SO₂NR₆R₇, wherein R₆ and R₇, together with the nitrogen atom to which they are attached, form a heterocyclic ring, wherein said heterocyclic ring is selected from piperidine and piperazine, wherein said heterocyclic ring is substituted with at least one R₈; and
R₈ is independently selected from C₁-C₆alkyl optionally substituted with OH, ONO₂, wherein at least one of said at least one R₈ comprises at least one ONO₂ moiety

In preferred embodiments, R₁ is methyl or ethyl; R₂ is H; R₃ is ethyl or *n-*propyl; and R₄ is ethyl or n-propyl. In some embodiments, R₁ is ethyl; R₂ is H; R₃ is n-propyl; and R₄ is n-propyl.

In some embodiments, said compound of Formula (I) is a compound selected from any one of compounds 1-10, below, or a pharmaceutically acceptable salt thereof:
Compound 1: (1-((3-(5-ethyl-4-oxo-7-propyl-4,5-dihydro-3H-pyrrolo[3,2-d]pyrimidin-2-yl)-4-propoxyphenyl)sulfonyl)piperidine-4,4-diyl)bis(ethane-2,1-diyl) dinitrate 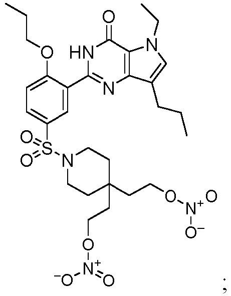
Compound 2: 2-(4-((3-(5-ethyl-4-oxo-7-propyl-4,5-dihydro-3H-pyrrolo[3,2-d]pyrimidin-2-yl)-4-propoxyphenyl)sulfonyl)piperazin-1-yl)ethyl nitrate; 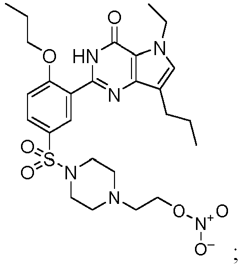
Compound 3: 2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethyl nitrate 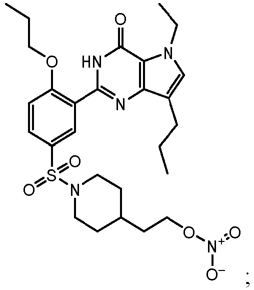
Compound 4: 3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)propyl nitrate 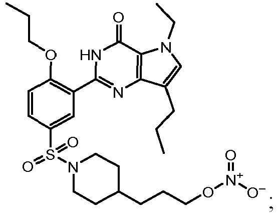
Compound 5: (R)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate 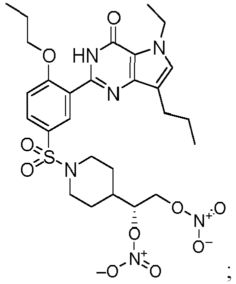
Compound 6: (S)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate 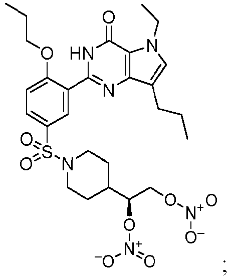
Compound 7: ((2R,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate 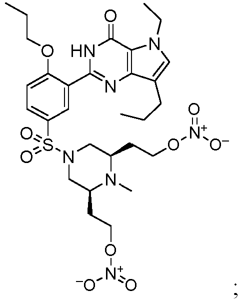
Compound 8: ((2S,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate 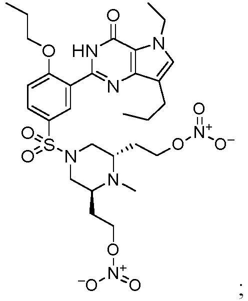
Compound 9: 3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-3-hydroxypentane-1,5-diyl dinitrate 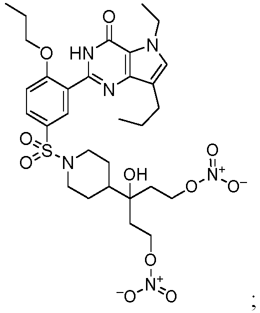 and
Compound 10: 2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-2-hydroxypropane-1,3-diyl dinitrate

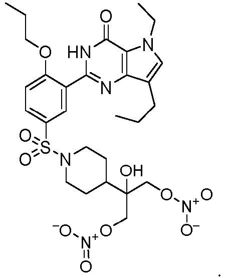

In some preferred embodiments, said compound of Formula (I) is (1-((3-(5-ethyl-4-oxo-7-propyl-4,5-dihydro-3H-pyrrolo[3,2-d]pyrimidin-2-yl)-4-propoxyphenyl)sulfonyl)piperidine-4,4-diyl)bis(ethane-2, 1-diyl) dinitrate (Compound 1), or a pharmaceutically acceptable salt thereof.

In some preferred embodiments, said compound of Formula (I) is 2-(4-((3-(5-ethyl-4-oxo-7-propyl-4,5-dihydro-3H-pyrrolo[3,2-d]pyrimidin-2-yl)-4-propoxyphenyl)sulfonyl)piperazin-1-yl)ethyl nitrate (Compound 2), or a pharmaceutically acceptable salt thereof.

In some preferred embodiments, said compound of Formula (I) is 2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethyl nitrate (Compound 3), or a pharmaceutically acceptable salt thereof.

In some preferred embodiments, said compound of Formula (I) is 3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)propyl nitrate (Compound 4), or a pharmaceutically acceptable salt thereof.

In some preferred embodiments, said compound of Formula (I) is (R)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate (Compound 5), or a pharmaceutically acceptable salt thereof.

In some preferred embodiments, said compound of Formula (I) is (S)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate (Compound 6), or a pharmaceutically acceptable salt thereof.

In some preferred embodiments, said compound of Formula (I) is ((2R,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate (Compound 7), or a pharmaceutically acceptable salt thereof.

In some preferred embodiments, said compound of Formula (I) is ((2S,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate (Compound 8), or a pharmaceutically acceptable salt thereof.

In some preferred embodiments, said compound of Formula (I) is 3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-3-hydroxypentane-1,5-diyl dinitrate (Compound 9), or a pharmaceutically acceptable salt thereof.

In some preferred embodiments, said compound of Formula (I) is 2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-2-hydroxypropane-1,3-diyl dinitrate (Compound 10), or a pharmaceutically acceptable salt thereof.

### Uses of the Compositions

In one aspect, the present disclosure provides PLGA microparticle-containing microneedles, preferably dissolvable microneedles, more preferably dissolvable candlelit microneedles, for use in a method of preventing or treating hair loss, or promoting hair growth, in a subject in need thereof. Any reference to a microneedle as described herein for use in a method of treating disease or disorder in a subject is also understood to include corresponding (i) methods of treating the disease or disorder in a subject in need thereof using the microparticle; (ii) use of the microparticle in the manufacture of a medicament for treating the disease or disorder; and (iii) use of the microparticle for treating the disease or disorder.

Without wishing to be bound by theory, the use of microneedles as described herein enables the administration of PLGA microparticles and compounds of Formula (I) directly to the site of action (preferably the dermis of the skin). Accordingly, in preferred embodiments, the use of the inventive microneedles and microneedle arrays avoids systemic exposure of compounds of Formula (I). In some embodiments, less than 50% of the compound of Formula (I) (preferably Compound 1) administered to a subject using the inventive microneedles enters the bloodstream of a subject. Preferably, less than 10% of the compound of Formula (I) (preferably Compound 1) enters the bloodstream of a subject, more preferably less than 1%, and most preferably less than 0.1% of the compound of Formula (I) (preferably Compound 1) administered to a subject using the inventive microneedles enters the bloodstream of a subject. In preferred embodiments, the amount of a compound of Formula I (e.g., Compound 1) in the bloodstream of a subject is substantially zero, e.g., is below the lower limit of quantitation (LLOQ) of the technique used to measure the concentration of the compound of Formula I (e.g., Compound 1) in the bloodstream of the subject.

In one aspect, the present disclosure provides a PLGA microparticle-containing microneedle as described herein for use in a method of preventing or treating hair loss, or promoting hair growth, preferably via:
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle.

In one aspect, the present disclosure provides a method of preventing or treating hair loss, or promoting hair growth, in a subject in need thereof, the method comprising administering to said subject an effective amount of a PLGA microparticle-containing microneedles as described herein, preferably wherein said preventing or treating hair loss, or promoting hair growth occurs via:
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle.

In one aspect, the present disclosure provides the use of a PLGA microparticle-containing microneedle as described herein in the manufacture of a medicament for preventing or treating hair loss, or promoting hair growth in a subject in need thereof, preferably wherein said preventing or treating hair loss, or promoting hair growth occurs via:
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle.

In one aspect, the present disclosure provides the use of a PLGA microparticle-containing microneedle as described herein for preventing or treating hair loss, or promoting hair growth in a subject in need thereof, preferably wherein said preventing or treating hair loss, or promoting hair growth occurs via:
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle.

In some embodiments, said hair loss is androgenetic alopecia (AGA) or chemotherapy-induced alopecia (CIA) or alopecia areata.

In preferred embodiments, said preventing or treating hair loss or promoting hair growth comprises (i) inducing anagen of hair follicles, (ii) promoting melanogenesis, (iii) increasing the number of hair follicles, (iv) increasing skin thickness (including the development of skin tissues), (v) promoting angiogenesis, (vi) proliferating cells in outer root sheath, and/or (vii) enhancing blood flow in the hair follicle in a subject with hair loss.

In some embodiments, the hair loss may be due to androgenetic alopecia (AGA). In some embodiments, the hair loss may be due to male pattern baldness or female pattern baldness. In another embodiment, the hair loss may be the result of skin damage, and for example, the hair loss may be hair loss caused by drug or chemotherapy treatment.

In some embodiments, the hair loss is scarring (or in another embodiment, scar) hair loss, e.g., due to scarring of the scalp area. The scarring hair loss typically occurs on the top of the scalp and is typically observed in women. Scarring can be the result of hair treatments, e.g., tight braiding, or can appear in postmenopausal women associated with inflammation of the hair follicles and the resulting scarring.

In some embodiments, the hair loss includes known baldness. In some embodiments, the baldness is caused by alopecia areata as an autoimmune disorder that causes uneven hair loss or shows a large area of hair loss in the form of an "island" of hair remaining from extensive hair loss.

In some embodiments, the hair loss includes hair loss due to compulsive hair pulling.

In some embodiments, the hair loss is due to triangular alopecia, typically characterized in that hair in the temporal lobe region begins to fall off in childhood. In some embodiments, hair loss may be completely progressed, or some fine and thin hair may remain.

In some embodiments, the hair loss includes hair loss due to telogen alopecia.

In some embodiments, the hair loss is caused by loose-anagen syndrome.

In some embodiments, the hair loss is caused by fungal infection, e.g., tinea capitis (scalp ringworm) which is characterized by patches of dandruff and spreads from the scalp to cause hair breakage, redness, swelling and exudation.

In some embodiments, the hair loss includes hair loss which is associated with certain conditions such as cancer, thyroid disease, protein deficiency in the diet or low serum iron levels, or associated with certain environmental stimuli such as chemotherapy or other radiotherapy.

In one aspect, the present disclosure provides PLGA microparticle-containing microneedles for use in a method of treating any disease, disorder or symptom associated with baldness.

In one aspect, the present disclosure provides PLGA microparticle-containing microneedles for use in a method of treating a degenerative skin disorder. In one aspect, the present disclosure provides PLGA microparticle-containing microneedles for use in a method of treating any disease, disorder or symptom associated with alopecia.

Methods for determining the presence and severity of hair loss as described herein are well known in the art.

In some embodiments, the hair loss is hair loss on the scalp of the subject. In some embodiments, the hair loss is hair loss of the eyebrow of the subject. In some embodiments, the hair loss is hair loss in the subject's scarred skin tissue, which may be the subject's scalp, eyebrows, arms or legs. In another embodiment, any other area or region of the skin where there is body hair may be treated by the method of the present invention.

In one aspect, the present disclosure provides PLGA microparticle-containing microneedle as described herein for use in a method of treating a disease or disorder in a subject in need thereof, preferably wherein said disease or disorder is selected from a skin disease, skin aging, steroid-induced skin atrophy, and erectile dysfunction.

In one aspect, the present disclosure provides a method of treating a disease or disorder in a subject in need thereof, preferably wherein said disease or disorder is selected from a skin disease, skin aging, steroid-induced skin atrophy, and erectile dysfunction, the method comprising administering to the subject an effective amount of PLGA microparticle-containing microneedles as described herein.

In one aspect, the present disclosure provides the use of a PLGA microparticle-containing microneedle as described herein in the manufacture of a medicament for the treatment of a disease or disorder, preferably wherein said disease or disorder is selected from a skin disease, skin aging, steroid-induced skin atrophy, and erectile dysfunction.

In one aspect, the present disclosure provides the use of a PLGA microparticle-containing microneedle as described herein for the treatment of a disease or disorder in a subject in need thereof, preferably wherein said disease or disorder is selected from a skin disease, skin aging, steroid-induced skin atrophy, and erectile dysfunction.

In some embodiments, said disease or disorder is a skin disease. In some embodiments, said disease or disorder is skin aging or steroid-induced skin atrophy. In some embodiments, said disease or disorder is atopic dermatitis. In some embodiments, said disease or disorder is arthritis. In some embodiments, said disease or disorder is rheumatology. In some embodiments, said disease or disorder is skin wrinkles (i.e., rhytids). In some embodiments, said disease or disorder is skin aging. In some embodiments, said disease or disorder is steroid-induced skin atrophy. In some embodiments, said disease or disorder is erectile dysfunction.

In the present invention, the PLGA microparticle-containing microneedles, preferably dissolvable candlelit microneedles or the array comprising said microneedles, preferably said dissolvable candlelit microneedles are administered to said subject once or twice within 30 days. In some embodiments, the microneedle, preferably dissolvable candlelit microneedle or array comprising said microneedles, preferably dissolvable candlelit microneedle is administered to said subject about three times every day. In some embodiments, the microneedle, preferably dissolvable candlelit microneedle or array comprising said microneedles, preferably dissolvable candlelit microneedle is administered to said subject about once every day. In some embodiments, the microneedle, preferably dissolvable candlelit microneedle or array comprising said microneedles, preferably dissolvable candlelit microneedle is administered to said subject about once every 7 days. In some embodiments, the microneedle, preferably dissolvable candlelit microneedle or array comprising said microneedles, preferably dissolvable candlelit microneedle is administered to said subject about once every 15 days. In preferred embodiments, the microneedle, preferably dissolvable candlelit microneedle or array comprising said microneedles, preferably dissolvable candlelit microneedle is administered to said subject about once every 30 days. In some embodiments, the microneedle, preferably dissolvable candlelit microneedle or array comprising said microneedles, preferably dissolvable candlelit microneedle is administered to said subject about once every 60 days. In some embodiments, the microneedle, preferably dissolvable candlelit microneedle or array comprising said microneedles, preferably dissolvable candlelit microneedle is administered to said subject about once every 90 or more days.

### Examples

The disclosure is further illustrated by the following examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

Unless otherwise indicated, all solvents and reagents were purchased from commercial sources and used without further purification.

PLGA was purchased from Sigma Aldrich (Resomer^{®} RG502, lactide:glycolide 50:50, ester terminated, MW: 7000-17000 Da, inherent viscosity: 0.16-0.24 dL/g, 0.1 % (w/v) in chloroform (25 °C, Ubbelohde) (size 0c glass capillary viscometer), form: amorphous, transition temperature: Tg 42-46 °C, Supplier: Sigma Aldrich. Tris Hydrochloride was obtained from Carl Roth GmbH. Polyvinyl alcohol (Mowiol^{®} 8-88, Mw: ~ 67000 Da) was obtained from Sigma Aldrich. Dichloromethane (DCM) was obtained from Carl Roth GmbH. Ultrapure water was produced in-house. Acetone (technical grade) was obtained from Carl Roth GmbH. Dulbecco's Phosphate Buffered Saline (D8537-1L) was obtained from Sigma Aldrich. Human blood plasma was obtained from Blutspendezentrum Zürich. Normocin was obtained from Invivogen. Methanol (LCMS grade) was obtained from VWR. Acetonitrile (LCMS grade) was obtained from Supelco^{®}. Isopropanol (LCMS grade) was obtained from VWR Formic acid (98-100%) was obtained from Merck. Ammonium formate (≥ 99.0%; Cat. No. 70221) was obtained from Merck.

Synthesis of Compounds of represented by Formula (I): Compounds of Formula (I) as well as their syntheses are disclosed in WO/2018/215433, the disclosure of which is incorporated by reference in its entirety. In particular, Examples 106 and 20 of WO/2018/215433 describe the preparation of preferred compounds 1n and 1i, respectively. In the Examples below, Compound 1 was prepared by Aragen Life Sciences (purity 99.41%).

### Example 1: Preparation of PLGA Microparticles (lactide:glycolide 50:50) Loaded with Compound 1

Preparation of Tris hydrochloride buffer-polyvinyl alcohol solution
50 g polyvinyl alcohol and 151.4 g Tris HCl were added to 4500 ml pure water, heated at 70°C and stirred until both were completely dissolved. After cooling to room temperature, the solution was adjusted to a pH 9 by adding NaOH 6 M solution and filled up to 5000 ml total volume with pure water to yield a 1% polyvinyl alcohol (w/v)-TRIS hydrochloride buffer solution.

### Preparation of PLGA microparticles (lactide:glycolide 50:50) Loaded with Compound 1

In a beaker, 90 ml of the 1% polyvinyl alcohol-Tris hydrochloride buffer solution was stirred at 700 rpm. A beaker containing 200 mg PLGA and 10 mg Compound 1 (5 % theoretical loading) was precooled on ice for 5 min, then 1 ml of ice-cold dichloromethane was transferred into the precooled beaker and mixed for 2 min at 20000 rpm with a homogenizer. 10 ml of the 1% polyvinyl alcohol-Tris hydrochloride buffer solution was added and the mixture was stirred for an additional 2 min at 20000 rpm with the homogenizer. Immediately after the mixing process, the solution was transferred into the beaker containing 90 ml of 1% polyvinyl alcohol-Tris hydrochloride buffer solution and the solution was stirred on the magnetic stirrer at 700 rpm for 18h to allow the dichloromethane to evaporate. After the evaporation of dichloromethane, the resulting aqueous suspension of PLGA microparticles was washed 3 times with ultrapure water and the microparticles thus formed were collected by centrifuging the suspension at max. 2500 rpm. The flask containing the PLGA microparticles was attached to a rotation evaporator and cooled to -80°C in a Dewar vessel containing acetone and dry ice. The process was complete when the water-microparticles suspension was frozen to the surface of the flask. The frozen water-microparticles mixture was stored over 30 minutes in a -80°C freezer. The mixture was then freeze dried by placing the flask in a freeze dryer and drying over a period of 24 to 48 h. The PLGA microparticles were stored in airtight containers at -20°C.

### Example 2: Preparation of the PLGA Microparticles Vehicle Without Loading a Compound of Formula (I)

In a beaker, 90 ml of 1% polyvinyl alcohol-Tris hydrochloride buffer solution (prepared as described in Example 1) was stirred at 700 rpm. A beaker containing 200 mg PLGA was precooled on ice for 5 min, then 1 ml of ice-cold dichloromethane was transferred into the precooled beaker and mixed for 2 min at 20000 rpm with a homogenizer. 10 ml of the 11% polyvinyl alcohol-Tris hydrochloride buffer solution was added and the mixture was stirred for an additional 2 min at 20000 rpm with the homogenizer. Immediately after the mixing process, the solution was transferred into the beaker containing 90 ml of 1% polyvinyl alcohol-Tris hydrochloride buffer solution and the solution was stirred on the magnetic stirrer at 700 rpm for 18h to allow the dichloromethane to evaporate. After the evaporation of dichloromethane, the resulting aqueous suspension of PLGA microparticles was washed 3 times with ultrapure water and the microparticles thus formed were collected by centrifuging the suspension at max. 2500 rpm. The flask containing the PLGA microparticles was attached to a rotation evaporator and cooled to -80°C in a Dewar vessel containing acetone and dry ice. The process was complete when the water-microparticles suspension was frozen to the surface of the flask. The frozen water-microparticles mixture was stored over 30 minutes in a -80°C freezer. The mixture was then freeze dried by placing the flask in a freeze dryer and drying over a period of 24 to 48 h. The PLGA microparticles were stored in airtight containers at -20°C.

### Example 3: Preparation of Compound 1-loaded PLGA microparticles (Lactide:Glycolide 65:35)

PLGA microparticles are prepared using 200 mg of PLGA lactide:glycolide (65:35 wt/wt) and 10 mg Compound 1 (5 % theoretical loading) using a procedure analogous to that described in Example 1.

### Example 4: Characterization of PLGA Microparticles

A laser diffractometer (Sympatec Helos H1964 PSD) and a cuvette (6mL; Sympatec Helos) were used to measure the size and the particle scattering diffusometry (PSD) of the PLGA microparticles. Particle diameter was calculated using software provided by the manufacturer.

The measurements were carried out using a Helos R1 lens with a range of 0.1 - 35 µm and an R3 lens with a range of 0.9 - 175 µm. Dried PLGA microparticles were first dispersed in water containing 0.1 % Tween-80 and placed in an ultrasonic bath for 1 min. Each sample was measured three times, and the average of these measurements was calculated. The designations "x10", "x50", and "x90" refer to the diameters of the particles at the tenth percentile, the 50^{th} percentile, and the 90^{th} percentile of total particle size, respectively.

Two mg of the dried PLGA microparticles were dissolved in 1 mL acetonitrile, diluted, and measured with liquid chromatography - mass spectrometry (LC-MS) to confirm inclusion of Compound 1 in the microparticles.

Drug loading was calculated according to Equation 1, and encapsulation efficiency was calculated according to Equation 2. Equation 1: Theoretical drug loading of PLGA microparticles Drug Loading [%] = (Drug within microparticles [mg])/(Total mass of PLGA [mg])*100 Equation 2: Efficiency of Encapsulation of Drug in of PLGA microparticles Encapsulation Efficiency [%]=(Drug within microparticles [mg])/(initial drug amount [mg])*100

The microparticles produced according to Examples 1 were found to have a mean size distribution of 4.36 ± 0.05µm.

The theoretical loading of Compound 1 in the PLGA microparticles as described in Example 1 was 5.10±0.50%. The encapsulation efficacy was 10.31±0.97%. Accordingly, the total loading of Compound 1 in the PLGA microparticles of Example 1 was 0.5% (w/w).

### Example 5: In vitro release assay of Compound 1 from PLGA microparticles (lactide:glycolide 50:50) loaded with Compound 1

Preparation of human blood plasma - buffer Simulated Interstitial fluid (SISF) solution

A buffer (996 mL) containing NaCl (117 nM), KCl (3 nM) CaCl₂ x 2H₂O (2.8 nM), MgCl₂ (1 nM), NaHCO₃ (27 nM), Na₂SO₄ (0.5 nM), and K₂HPO₄ (1 nM) was mixed with 1000 ml human blood plasma and a 0.2% (w/w) solution of Normocin (4 mL) was added.

5 mg dried PLGA microparticles, prepared as described in Example 1, were placed into the donor chamber of the "rapid equilibrium dialysis" assay plate (Thermo Scientific). To the donor chamber was added 500 µl PBS.

750µL of the human blood plasma - SISF buffer solution, was spiked with the internal standard Compound 1 (D4), and added to the receiver chamber.

The RED plate was then sealed with an aluminum foil seal and placed in the incubator at 37 °C and shaken at 200 rpm dufor 696 hours.

For sampling at each time point, the entire volume of the receiver chamber was removed and refilled with fresh human blood plasma - SISF buffer solution. The samples were stored at -20°C until the last day of sampling at day 29. The samples were thawed at room temperature for 60 min for the LCMS measurement using the solid phase extraction (SPE, Protein precipitation Sirocco plate, Waters) method. The quantification of Compound 1 in the human blood plasma - SISF buffer solution was determined using a calibration curve prepared by measuring the amount of deuterated internal standard Compound 1-D4. The calibration curve was prepared at 6 calibration concentrations of 0.1 nm, 0.5 nM, 1.0 nM, 5.0 nM, 10 nM, 25 nM and 50 nM, Compound 1, each of which was spiked with 1nM deuterated Compound 1-D4 as an internal standard.

**LCMS method:** Agilent 1100 quaternary pump mass spectrometry AB Sciex API 4000 LC/MS/MS Triple Quad. Injection volume: 25 µl, flow rate 400 µl/min. ColumnCORE-25A-0502U, ACE UltraCore 2.5 SuperC18, 50 x 2.1 mm. Column oven temperature 40 °C. Solvent A: Acetonitrile / MeOH 50:50 v/v % + 0.1 % fumaric acid, 2 mM ammonium formate. Solvent B: Water + 0.1 % fumaric acid, 2mM ammonium formate.

LC method gradient

| Time [min] | Solvent A [%] | Solvent B [%] |
|---|---|---|
| 1.50 | 70 | 30 |
| 2.00 | 5 | 95 |
| 5.00 | 5 | 95 |
| 5.50 | 95 | 5 |
| 6.20 | 95 | 30 |
| 6.50 | 70 | 30 |

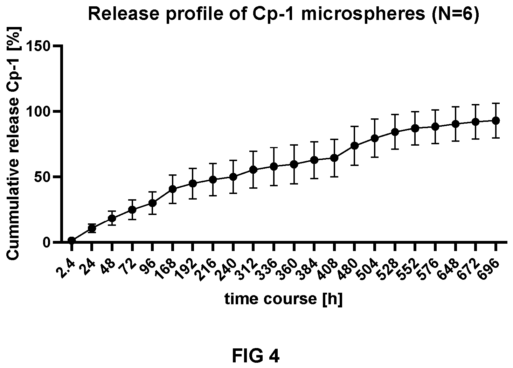

As the PLGA microparticle were degraded, the encapsulated Compound 1 was released over the course of 29 days. FIG 4 is a plot of the cumulative release of Compound 1 over the 29-day course of measurement. The final release profile was 88.16 ± 5.62% on Day 29, confirming that most of the M119 was released from the PLGA microsphere once dissolved in the simulated interstitial fluid.

### Example 6: Preparation of Candlelit Microneedles Loaded with PLGA Microparticles Comprising Compound 1

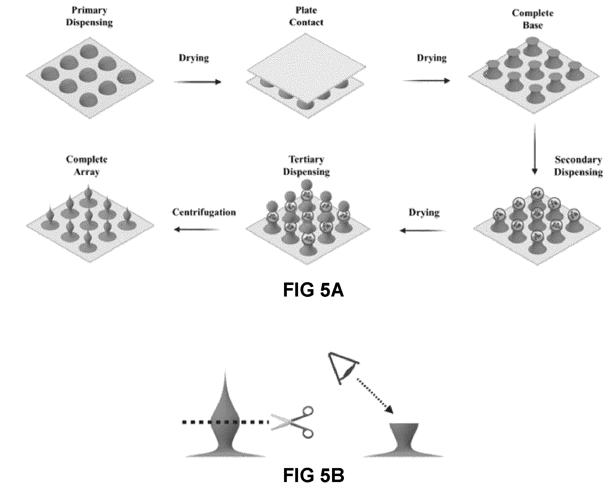

Candlelit microneedles were prepared as described below and as shown in the schematic in FIG 5. The fabrication of CMN consisted of three main steps: primary dispensing to create the base layer, secondary dispensing to create the core layer, and tertiary dispensing and centrifugation to create the tip layer.

### Hyaluronic Acid Solution

A 60% (w/v) solution of pharmaceutical-grade hyaluronic (PHA, 30 kDa; Uscarepharm, Suwon, Republic of Korea) was prepared by dissolving 600 mg PHA in 1000 mg PBS buffer (pH 7.4) using a paste mixer.

### Base Layer

Thirty-seven (37) droplets of the 60% (w/v) solution of pharmaceutical-grade hyaluronic acid in phosphate buffer were dispensed on a polyurethane patch, using an air compression dispenser. The distance between droplets was 1.5 mm. A plate cover was then placed on top of the dispensed solution droplets, with a 250-µm gap between the patch and cover. The dispensed solution droplets touched the plate cover and stayed connected with the plate. The solution droplets were dried at room temperature overnight, forming an hourglass shape. The plate cover was then removed, leaving the completed base structures.

### Core Layer

The core solution was formed by mixing 1000 mg PBS buffer (pH 7.4) with 600 mg PHA and 30 mg PLGA microparticles encapsulating Compound 1. The microparticles comprised 0.5% (w/w) Compound 1, for a total mass of 0.15 mg Compound 1 in the 30 mg of PLGA microparticles.

The total amount of Compound 1 within the core layer solution was therefore 0.0092% (w/w), as shown in Equation 3: Percentage (w/w) of Compound 1 in Core Layer Solution (0.15 mg Compound 1)/(1000 mg PBS + 600 mg PHA + 30 mg PLGA) = 0.0092%.

The core solution (8.3 mg total, i.e., 0.76 µg Compound 1) was dispensed on top of the dried base layer to form the core layer array. Accordingly, the total mass of Compound 1 in the array of 37 microneedles was 0.76 µg Compound 1 (0.0205 µg Compound 1 per core layer of microneedle).

After the core solution was deposited, the core layer was dried to remove the PBS. The resulting core layer (after drying) thus comprised 0.0238% (w/w) Compound 1 as shown in Equation 4: Percentage (w/w) of Compound 1 in Core Layer (0.15 mg Compound 1)/(600 mg PHA + 30 mg PLGA) = 0.0238%.

### Tip Layer

Next, the tip of the MP-CMN was fabricated by preparing a 30% (w/v) PHA solution in PBS buffer and dispensing it on top of the core using an air compression dispenser, followed by centrifugal lithography at 250 × g for 30 s. The final microparticle-containing candlelit microneedles contain 0.76 µg Compound 1 per 37-microneedle array; each array contains 37 MP-CMNs in total.

Morphological characteristics of the resulting candlelit microneedles were analyzed using bright field microscope (M165 FC; Leica, Wetzlar, Germany). MP-CMN height, width and tip diameter were observed and measured. CMN microparticle loading analysis: SEM image of sectional cut of MP-CMN was obtained by FE-SEM microscope (JEOL-7800F; JEOL, Tokyo, Japan). The height of the microneedles was found to be around 1000 µm; the width between 250 µm and 450 µm, and the tip diameter from about 20 µm to about 50 µm.

The resulting complete microneedle array comprised 37 microneedles and a total dry mass of 6.8 mg. A total of 0.76 µg of Compound 1 was present in the microneedle array. Accordingly, the microneedles comprised 0.011% Compound 1. The core layers of the microneedles comprised 0.0238% as given in Equation 4 above.

### Example 7: Preparation of Compound 1-loaded candlelit microneedle (CMN)

CMN loaded with Compound 1 are fabricated using HA as in Example 6, PLGA microparticles (lactide:glycolide 50:50) containing 2% drug loading amount of Compound 1 and the respective 0.952% of Compound 1 in the core layer.

The following high performance liquid chromatography (HPLC) conditions for quantitative analysis of Compound 1 loading in the CMN are used: A reverse-phase HPLC (Waters 600S; Waters, Milford, MA, USA) with a C18 column (150 mm × 4.6 i.d., Cosmosil 5C18-AR-II; Nacalai Tesque Inc., Kyoto, Japan). To set a calibration curve, a preparation of a stock solution of Compound 1 is sequentially diluted to prepare a range of concentrations from 1.5 mg/mL to 0 mg/mL (R² ≥ 0.99). A mobile phase is prepared with 50% (v/v) acetonitrile and 50% (v/v) distilled water and the flow rate is set at 1 mL/min. The detection wavelength of Compound 1 is 254 nm. The retention time is determined to be about 6.625 min.

### Example 8: Preparation of rhodamine B-loaded candlelit microneedle (CMN)

Candlelit microneedles were prepared as described below and as shown in the schematic in FIG 5. The fabrication of CMN consisted of three main steps: primary dispensing to create the base layer, secondary dispensing to create the core layer, and tertiary dispensing and centrifugation to create the tip layer.

### Hyaluronic Acid Solution

A 60% (w/v) solution of pharmaceutical-grade hyaluronic (PHA, 30 kDa; Uscarepharm, Suwon, Republic of Korea) was prepared by dissolving 600 mg PHA in 1000 mg PBS buffer (pH 7.4).

### Base Layer

Thirty-seven (37) droplets of the 60% (w/v) solution of pharmaceutical-grade hyaluronic acid in phosphate buffer were dispensed on a polyurethane patch, using an air compression dispenser. The distance between droplets was 1.5 mm. A plate cover was then placed on top of the dispensed solution droplets, with a 250-µm gap between the patch and cover. The dispensed solution droplets touched the plate cover and stayed connected with the plate. The solution droplets were dried at room temperature overnight, forming an hourglass shape. The plate cover was then removed, leaving the completed base structures.

### Core Layer

The core solution was formulated by mixing 1000 mg PBS buffer (pH 7.4) with 600 mg PHA. 0.01 ml of a solution of rhodamine B, (concentration of 1% (w/w)) was added to the core solution. The core solution was dispensed on top of the dried base layer to form the core layer array. After the core solution was deposited, the core layer was dried to remove the PBS.

### Tip Layer

Next, the tip of the MP-CMN was fabricated by preparing a 30% (w/v) PHA solution in PBS buffer and dispensing it on top of the core using an air compression dispenser, followed by centrifugal lithography at 250 × g for 30 s.

### Example 9: Insertion Analysis and Dissolution of Candlelit Microneedles on Pig Cadaver Skin

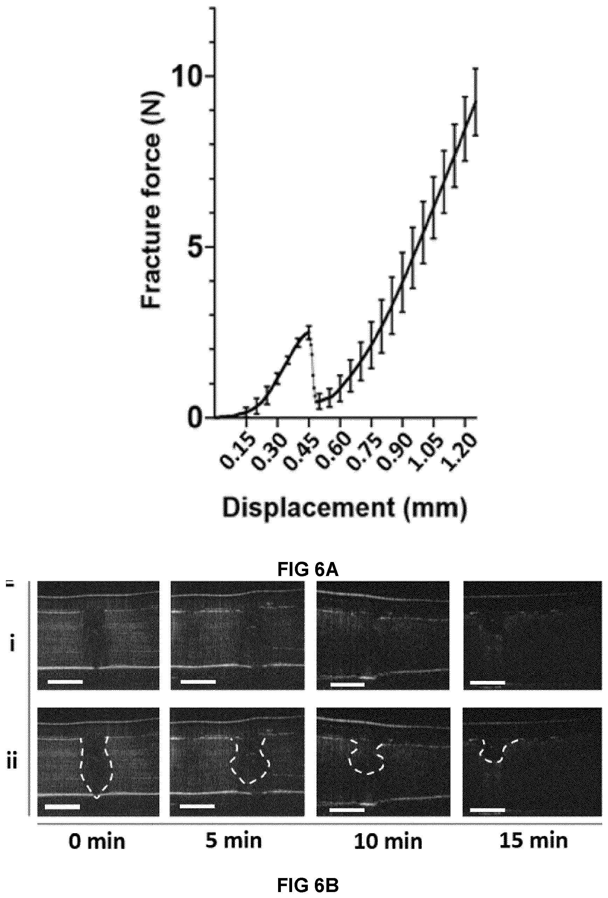

The insertion capability (i.e., skin penetrating capability) of the candlelit microneedles loaded with microparticles comprising Compound 1 was tested *in vitro* using animal skin tissue. Mechanical strength of the CMNs was measured using a force analyzer (Z0.5TN; Zwick/Roell Inc., Ulm, Germany). The probe of the force analyzer measured the vertical axial strength of the MP-CMN by pressing the microneedle at a vertical velocity of 3.0 mm/min. MP-CMN mechanical strength was first measured to analyze the maximum pressure force that each MP-CMN could withstand before breakage. As shown in FIG 6A, single MP-CMN was able to withstand 2.37 ± 1.23 N of pressure force before breaking, which is sufficient enough to be inserted into human skin and retain its shape.

MP-CMN morphological characteristics were visualized using a bright-field microscope (M165 FC; Leica, Wetzlar, Germany). The height, width, and tip diameter of the MP-CMN were subsequently measured. The height of the microneedles was found to be around 1000 µm; the width between 250 µm and 450 µm, and the tip diameter from about 20 µm to about 50 µm.

The skin penetration ability and insertion depth of MP-CMN was evaluated by applying it to porcine cadaver skin (Cronex, Hwaseong, Korea) and analyzing sectional cuts using optical coherence tomography (OQ StrataScope 1.0; Lumedica, Durham, NC, USA). Scanning electron microscopic (SEM) images of the section-cut MP-CMN were obtained using an FE-SEM (JEOL-7800F; JEOL, Tokyo, Japan).

Although single MP-CMN was capable of withstanding the physical force needed to penetrate the skin, the number of microneedles on a single patch also determine insertion quality. Pig cadaver skin was selected for its similar histological and physiological properties to human skin, suggesting that it is a good model for human skin permeability. To observe the successful insertion of a complete CMN array, rhodamine B was loaded in the core layer of CMN for visualization as described in Example 8 and inserted onto pig cadaver skin with 2 mm thickness for analysis of overall insertion quality. A cross-sectional image of an inserted row of candlelit microneedles measured by fluorescence microscopy confirmed that the core layers of the CMNs were delivered to the pig cadaver skin. The tip layer of the microneedle penetrated to a final depth of 700 µm to 800 µm. The core layer comprising the microparticles reached a depth of 250 µm to 500 µm. Such results demonstrate that the base layer had provided enough support to allow the core layer to penetrate into the skin to a therapeutically relevant depth.

The insertion was also observed with optical coherence tomography (OCT) imaging analysis for observation of MP-CMN dissolution in pig cadaver skin after insertion. The fluorescence measurement did not depict the dissolution rate of CMN, as rhodamine B is quantified regardless the CMN dissolution. OCT imaging analysis allows the observation of CMN size at different time points. As shown in FIG 6B, MP-CMN was continuously viewed through OCT and images were captured at 5 minute intervals to track the shape change post insertion into pig cadaver skin. At the 15-minute timepoint, the OCT image showed remaining of MP-CMN base layer, which measured around 250 µm.

Although the data of MP-CMN insertion analysis supports robust insertion into animal skin model, it is important to note that the dissolution of MP-CMN after insertion does not imply immediate release of Compound 1 encapsulated inside the PLGA microparticles. Since Compound 1 is encapsulated by the PLGA microparticles, the release of Compound 1 was not determined by the dissolution of the MP-CMN, but rather by the degradation of the PLGA microparticles originally present in the core layer thereof. The degradation of the Compound 1-encapsulating PLGA microparticles functions as a method for sustained release of Compound 1.

For correlation of *in vitro* insertion experimental data with *in vivo, in vivo* application on live C57BL/6 male mice were conducted to test whether MP-CMN shows effective insertion on live animals. Patches of CMNs containing rhodamine B in the core layer were inserted into the dorsal skin of live mice before and after hair removal to investigate whether the CMN array could successfully penetrate mice skin regardless of hair presence. The base layer of the CMNs did not contain rhodamine B to demonstrate the delivery of core layer into mouse dorsal skin.

The CMN patches were removed 15 minutes after insertion into mouse dorsal skin. The remnants of the CMNs on the removed patches showed no trace of rhodamine B, indicating that 15 minutes of insertion allowed full delivery of rhodamine B in core layer to the skin, while leaving the colorless base layer. The same results were observed whether or not the hair of the mice had been removed from the mouse dorsal skin prior to application of the CMNs, demonstrating that the CMNs can deliver the core layer to the skin with or without hair removal.

In contrast, an analogous in vivo experiment using conventional dissolvable microneedles (DMNs) demonstrated that the DMNs failed to deliver rhodamine B to the skin of unshaven C57BL/6 mice. A conventional DMN patch was applied and removed 15 minutes after insertion into mouse dorsal skin. Only small traces of the DMN microneedle patch application were visible, with no indications of rhodamine B. No fluorescence was observed following the DMN application, suggesting that rhodamine B was not delivered. Additionally, all DMNs were found to have bent ends, suggesting incomplete insertion due to the presence of hair on the mouse dorsal skin.

Without wishing to be bound by theory, the results suggest that hair growth induced by MP-CMN does not halt the administration of the core layer of CMPs to the skin.

### Example 10: Enhancement effect in injection of Compound 1 using microneedles

The *in vivo* efficacy of a microarray of dissolvable candlelit microneedles comprising microparticles encapsulating Compound 1 to promote hair growth was evaluated in male C57BL/6 mice. Mice (6 weeks old; Orient Bio, Seongnam, Republic of Korea) were kept for 1 week for adaptation to the environment. Then, the 7-week-old mice were shaved to remove the hair on dorsal skin area.

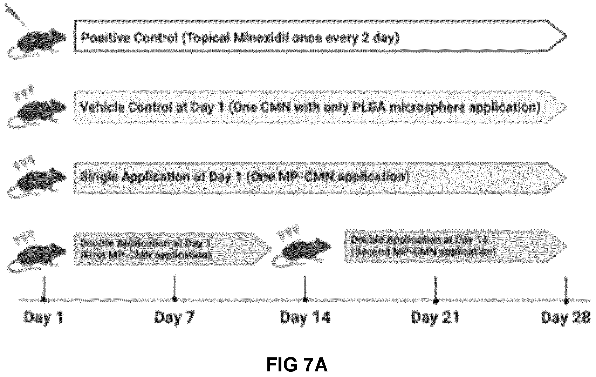

A schematic representation of the in vivo experimentation protocol is shown in FIG 7A. Five experimental groups were used with four mice in each group. Every mouse had its dorsal skin shaved prior to administration of agents. A negative control group was given no treatment on their dorsal skin throughout 28 days (not shown in FIG 7A). A positive control group received topical application of 2% w/v minoxidil once every 2 days. A vehicle control group was administered dissolvable candlelit microneedles loaded with PLGA microparticles not comprising a compound of Formula (I) as described in Example 2. A single application group and double application group each were administered candlelit microneedles which contained Compound 1 encapsulated in PLGA microparticles. The single application group received application on day 0, and the double application group received total 2 applications at day 0 and at day 14.

The 2% (w/v) minoxidil solution was prepared as 2% (w/v) solution in polyethylene glycol 400 (PEG 400; Sigma-Aldrich, St. Louis, MO, USA) and ethanol (99.5 %°; Sigma-Aldrich) at a ratio of 7:3 (v/v)

Hair growth was recorded on days 1, 7, 14, 21, and 28.

Histological staining: Mouse skin tissues were excised from the dorsal area and fixed overnight in 4% w/v paraformaldehyde (Sigma-Aldrich). The tissues were cut into 10 µm thick slices, which were then stained with hematoxylin and eosin (H&E) for analysis of skin thickness and hair regeneration. The stained slices were observed under bright field microscope (M165 FC; Leica).

Statistical analysis: Data were expressed as the means ± standard deviations. Statistical analysis was performed using Student's t-test or analysis of variance (ANOVA) test with GraphPad Prism 6.0 software (GraphPad Software Inc.). The results were obtained from four or more groups. Statistical significance was set at *p* ≤ 0.05 *(* p* < 0.05, ** *p* < 0.01, and **** p* < 0.001).

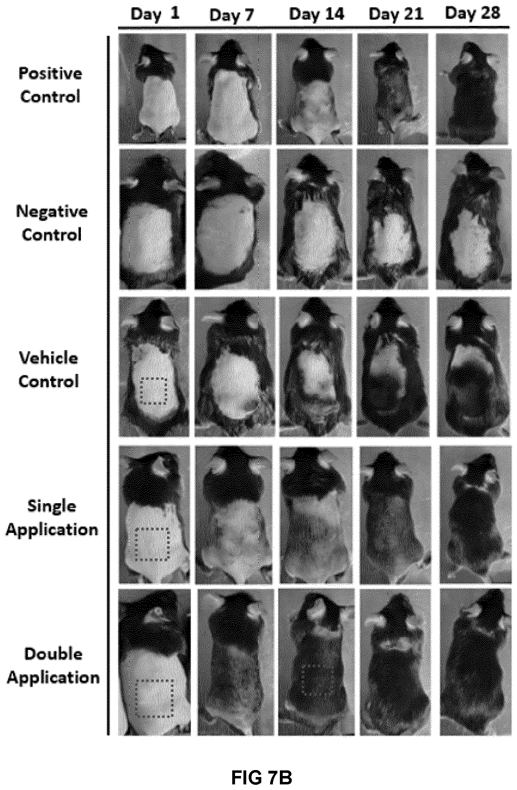

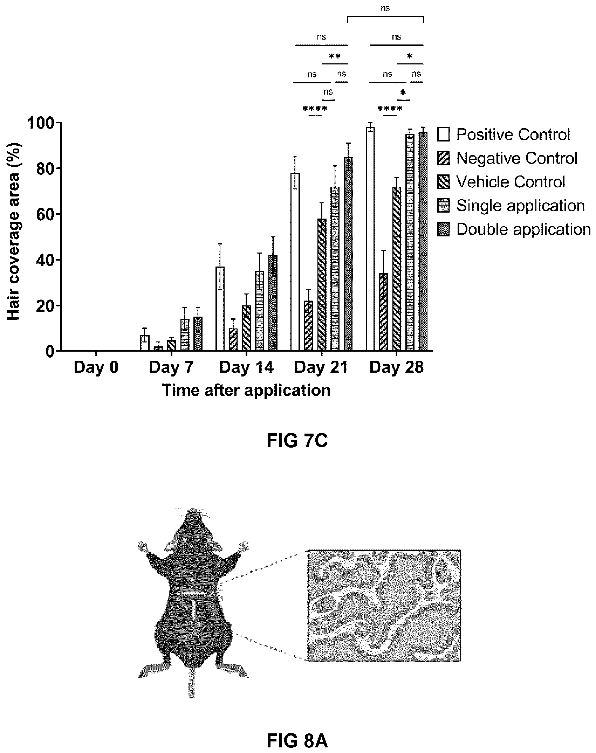

FIG 7B shows representative photos of the dorsal skin area of mice from each experimental group on days 1, 7, 14, 21, and 28. FIG 7C is a plot of the percent hair coverage area of the dorsal skin area analyzed by measuring the density of hair on days 0, 7, 14, 21 and 28. On day 7, the vehicle control, single and double application groups showed signs of hair growth. The vehicle control showed a limited area of hair recovery, the both single and double application groups had superior hair recovery. The positive control group started showing hair growth from day 14, while the negative group showed almost no signs of hair growth except for the outer edges of dorsal area. On day 21, both the positive control group and the single application group showed similar hair growth rate. The double application group had denser hair concentration compared to all other test groups. The vehicle control group also had a portion of its dorsal skin covered with hair, while also having naked parts. On day 28, the positive control group, single application group, and double application group had recovered their original hair condition, while the double application group had no significant difference compared day 21, because hair growth was faster in the double application group compared to the other groups.

The area of hair regrowth was measured by calculating the blackened area from the prior stage using image contrast analysis tool. The positive control group and both the single application group and double application group showed the highest hair recovery, i.e., 98.1 ± 1.9%, 94.7 ± 2.1%, and 95.6 ± 2.0% respectively at day 28.

The vehicle control group also showed 72.1% ± 4.1% of hair recovery area at day 28 despite the fact that the candlelit microneedles in the vehicle control group were not loaded with a compound of Formula (I). This vehicle control group was included for the analysis of possible hair growth effect from PLGA itself as it has been previously reported to show angiogenesis. As the vehicle control showed, application of CMNs containing PLGA microparticles which did not contain a compound of Formula (I) led to a 73.4% hair recovery area compared to positive control group and a 76.5% and 75.3% hair recovery area compared to single application group and double application group. In the negative control group, hair recovery amounted to 34.5 ± 9.5% on day 28. Without wishing to be bound by theory, although the vehicle control showed less efficacy than topical minoxidil or CMNs loaded with a compound of Formula (I), the incorporation of PLGA microparticle may have led to increased angiogenesis in the mice, enhancing regrowth for hair follicles.

Although single application group and double application group showed similar hair growth, the double application group showed more hair growth after the second CMN application, where the hair recovery was at 84.8 ± 6.1% on day 21, showing large area of hair covered area. On Day 21, the hair coverage area following double application of MP-CMN was 1.46-fold higher than vehicle control (CMN with PLGA microsphere only) which was significant. On day 21, no significant difference was seen between the single application group and the double application group, and by day 28 the difference between the two groups was negligible.

### Changes in skin thickness and hair thickness

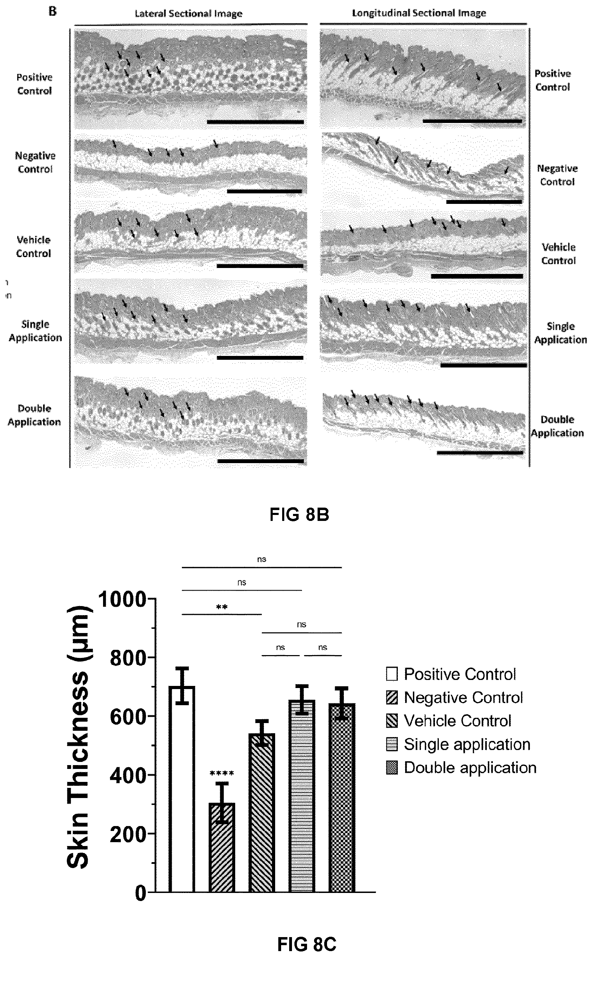

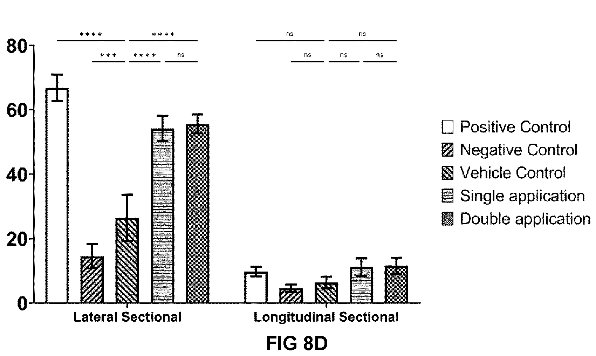

FIG 8 shows the results of the hematoxylin and eosin (H&E) staining analysis of the mouse dorsal skin excision. FIG 8A shows a schematic image of mouse skin lateral and longitudinal excision and a cross-sectional view.

FIG 8B shows cross sections of mouse dorsal skin on lateral side and longitudinal side for experimental groups. Cross section of hair in skin epithermal area is marked with black arrow in FIG 8B. FIG 8C shows average skin thickness of each experimental group, which is measured from the excised skin. The negative control had mean thickness of 305.13 ± 66.08 µm, which was significantly lower than other experimental groups. The positive control, vehicle control, single administration and double administration groups had mean thicknesses of 703.13 ± 59.02, 542.12 ± 40.87, 655.47 ± 46.85, and 643.46 ± 51.46 µm respectively, with no statistically significant differences between the groups. Such results support the hair recovery data, where at day 28, the positive control, single administration, and double administration groups all had similar hair growth rate as shown in FIG 7C.

Since the single administration group showed similar result when compared with the double administration group, the results suggest that a single CMN administration per 28 days is as potent as two administrations per 28 days. The number of hairs was counted to measure average number of hair cross section per 1 mm, both lateral and longitudinal cut, to show hair follicle growth progress (FIG 8D). The positive control, single administration, and double administration groups had average lateral hair per mm of 66.8 ± 4.1, 54.2 ± 3.9 and 55.6 ± 2.9 respectively. The vehicle control group had a thickness of 542.12 ± 40.87 and lateral hair per mm of 26.4 ± 7.1. The vehicle control group showed comparable growth indicators compared to the positive control, single administration and double administration groups despite having lower hair growth parameters in absolute terms.

As described above, specific parts of the content of the present invention are described in detail, and for those of ordinary skill in the art, it will be apparent that these specific descriptions are only preferred exemplary embodiments, and the scope of the present invention is not limited thereby. Accordingly, the substantial scope of the present invention will be defined by the appended claims and their equivalents.

## Claims

1. A dissolvable microneedle, wherein the dissolvable microneedle comprises:
(a) a dissolvable polymer; and
(b) a plurality of microparticles, wherein said microparticles comprise poly(lactic-co-glycolic) acid and a compound Formula (I) or a pharmaceutically acceptable salt thereof;
wherein said compound of Formula (I) has the structure: 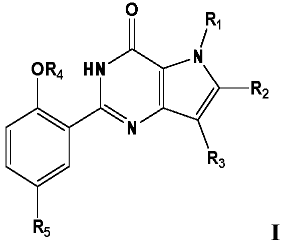 wherein
R₁ is C₁-C₃alkyl, preferably methyl or ethyl, further preferably ethyl; R₂ is H, CHO or CH=N-OH, preferably H, CHO or (E)-CH=N-OH, further preferably H;
R₃ is C₁-C₄alkyl, preferably ethyl or propyl, further preferably n-propyl;
R₄ is C₁-C₆alkyl, preferably ethyl or propyl, further preferably n-propyl;
R₅ is SO₂NR₆R₇, wherein R₆ and R₇, together with the nitrogen atom to which they are attached, form a heterocyclic ring, wherein said heterocyclic ring is selected from piperidine and piperazine, wherein said heterocyclic ring is substituted with at least one R₈; and
R₈ is independently selected from C₁-C₆alkyl optionally substituted with OH, ONO₂, wherein at least one of said at least one R₈ comprises at least one ONO₂ moiety.

2. The dissolvable microneedle of claim 1, wherein said dissolvable polymer comprises hyaluronic acid, collagen, polyvinylpyrrolidone, polylactic acid, poly(lactic-co-glycolic) acid, polyglycolic acid, polyethylene glycol, polycaprolactone, poly-amino acid, polypropylene fumarate, poly-gamma glutamic acid, polyvinyl alcohol, polyethyleneimine, albumin, dextran, fibrin, elastin, gelatin, alginic acid, polyacrylic acid, carboxymethylcellulose, and mixtures or copolymers thereof; preferably wherein said dissolvable polymer comprises hyaluronic acid.

3. The dissolvable candlelit microneedle of claim 1, wherein said dissolvable microneedle is a candlelit microneedle.

4. The dissolvable candlelit microneedle of claim 3, wherein said plurality of microparticles is dispersed within a core layer of said dissolvable candlelit microneedle.

5. The dissolvable microneedle of claim 4, wherein said core layer of said dissolvable candlelit microneedle comprises said microparticles at a concentration range from about 0.5% (wt/wt) to about 20% (wt/wt).

6. The dissolvable microneedle of any of the preceding claims, wherein said microparticle comprises poly(lactic-co-glycolic) acid, and wherein a ratio of lactic acid:glyclolic acid in said poly(lactic-co-glycolic) acid is from about 15:85 to about 85:15.

7. The dissolvable microneedle of any of the preceding claims, wherein said microparticles comprise said compound of Formula (I) at a concentration from about 0.01% (wt/wt) to about 5% (wt/wt).

8. The dissolvable microneedle of any of the preceding claims, wherein said dissolvable microneedle comprises said PLGA microparticles at a concentration range from about 0.25% (wt/wt) to about 10% (wt/wt).

9. The dissolvable microneedle of any of the preceding claims, wherein said dissolvable microneedle is arrayed on a microneedle supporting surface, wherein preferably said supporting surface is a patch.

10. The dissolvable microneedle of claim 1, wherein R₁ is methyl or ethyl; R₂ is H; R₃ is ethyl or n-propyl; and R₄ is ethyl or n-propyl.

11. The dissolvable microneedle of any of the preceding claims, wherein said compound of Formula (I) is Compound 1 or Compound 2: 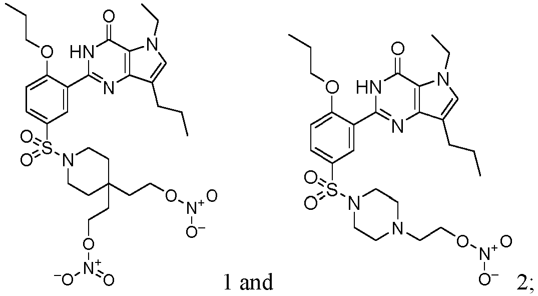 and wherein preferably said compound of Formula (I) is Compound **1;** or a pharmaceutically acceptable salt thereof.

12. The dissolvable microneedle of any of the preceding claims for use in a method of treating a disease or disorder in a subject in need thereof, preferably wherein said disease or disorder is selected from a skin disease, skin aging, steroid-induced skin atrophy, and erectile dysfunction.

13. The dissolvable microneedle for use according to claim 12, wherein the dissolvable microneedle is administered to said subject about once every 30 days.

14. A kit comprising:
(i) a microneedle as claimed in any one of claims 1-11, preferably wherein said microneedle is arranged in a microneedle array; and
(ii) an applicator for applying said microneedle array to the skin of a subject.

15. A method of preparing a dissolvable microneedle as described in any one of claims 1-11, the method comprising;
(i) encapsulating a compound of Formula (I) in a microparticle; and
(ii) incorporating said microparticle within said dissolvable microneedle.
